(19) Europäisches Patentamt
European Patent Office
Office européen des brevets

(11) **EP 3 649 938 A1**

(12) **EUROPEAN PATENT APPLICATION**
published in accordance with Art. 153(4) EPC

(43) Date of publication:
**13.05.2020 Bulletin 2020/20**

(51) Int Cl.:
***A61B 5/11*** *(2006.01)*

(21) Application number: **17916493.4**

(86) International application number:
**PCT/JP2017/024561**

(22) Date of filing: **04.07.2017**

(87) International publication number:
**WO 2019/008689 (10.01.2019 Gazette 2019/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventors:
• **HOTTA, Shinji**
**Kawasaki-shi**
**Kanagawa 211-8588 (JP)**
• **INOMATA, Akihiro**
**Kawasaki-shi**
**Kanagawa 211-8588 (JP)**

(74) Representative: **Haseltine Lake Kempner LLP**
**Lincoln House, 5th Floor**
**300 High Holborn**
**London WC1V 7JH (GB)**

(54) **INFORMATION PROCESSING DEVICE, INFORMATION PROCESSING SYSTEM, AND INFORMATION PROCESSING METHOD**

(57)    An information processing apparatus (100) acquires information regarding a stride length (SL) of a subject and information regarding angles ($\theta_L$ and $\theta_R$) of respective lower legs of the subject with respect to the ground in a case where both feet of the subject are grounded. The information processing apparatus (100) calculates angles ($\alpha$ and $\beta$) of respective knee joints of the subject and an angle ($\varphi$) of a hip joint of the subject that satisfy geometric constraints, derived from a structure of lower limbs of the subject in a case where both feet of the subject are grounded, based on the information regarding the stride length (SL) and the information regarding the angles ($\theta_L$ and $\theta_R$). For example, the geometric constraint is a constraint that is geometrically derived based on a (polygon 110) that is formed by using each thigh, each lower leg, and the ground as sides and the hip joint, each knee joint, and a contact point between each lower leg and the ground as vertices, by using the structure of the lower limbs of the subject.

FIG. 1

**Description**

TECHNICAL FIELD

The present invention relates to an information processing apparatus, an information processing system, and an information processing method.

BACKGROUND ART

Conventionally, in a medical field, it is required to grasp a condition of a patient in order to perform an appropriate medical treatment for the patient. For example, a walking form of the patient tends to be influenced by the degree of physical disorder due to a disease, the degree of recovery of a disease or injury, and the like, and there is a case where it is required to grasp the walking form of the patient in order to grasp the condition of the patient. For example, it is required to grasp whether or not the patient walks in an unnatural walking form, like without bending both legs, that applies a load to the patient's body and may cause an accident such as falling or a fall. Therefore, as an objective index of the patient's walking form, it is required to accurately calculate an angle of a knee joint of the patient and an angle of a hip joint of the patient and to enable to grasp the patient's walking form based on the angle of the knee joint of the patient and the angle of the hip joint of the patient. In particular, to enable to grasp the daily patient's walking form and grasp the patient's walking form outside the medical institution is preferable for applying appropriate medical care for the patient.

As the prior art, for example, there is a technology in which each measurement sensor is attached to surround each of the hip joints, the knee joints, and ankle joints of both legs of a pedestrian so as to obtain joint angles of the hip joints, the knee joints, and the ankle joints of the pedestrian based on the measurement data obtained by measuring an angular velocity and an acceleration at the time when the pedestrian is walking. Furthermore, for example, there is a technology that similarly attaches each measurement sensor, measures a change in the center of gravity and a change in the joint angle of the leg caused by walking, and calculates an index indicating a walking motion of a user based on the measured changes in the center of gravity and the joint angles and user's body information that is not changed by walking.

CITATION LIST

PATENT DOCUMENT

   Patent Document 1: Japanese Laid-open Patent Publication No. 2014-208257
   Patent Document 2: Japanese Laid-open Patent Publication No. 2012-65723

SUMMARY OF INVENTION

TECHNICAL PROBLEM

There is a case where it is required to cause a patient to wear a sensor device, measure an angle of a knee joint of the patient and an angle of a hip joint of the patient based on time-series data obtained from the sensor device, and grasp a walking form of the patient. However, in the prior art, a large number of measurement sensors have been required to accurately measure the angles of the knee joint and the hip joint of the patient. For example, it has been required to attach the sensor devices to various parts of lower limbs of the patient. Therefore, problems have occurred in that a high physical load and a high psychological load are applied to the patient when wearing the sensor and a measurement cost is high, for example, and it has been difficult to continuously perform the measurement on a daily basis.

In one aspect, an object of the present invention is to provide an information processing apparatus, an information processing system, and an information processing method that can accurately calculate an angle of a knee joint of a subject or an angle of a hip joint of the subject by using a small number of sensors.

SOLUTION TO PROBLEM

According to one aspect, an information processing apparatus, an information processing system, and an information processing method are proposed that acquire information regarding a stride length of a subject and information regarding an angle of each lower leg of the subject with respect to a ground in a case where both feet of the subject are grounded, and calculate at least any one of an angle of each knee joint of the subject and an angle of a hip joint of the subject that satisfy a geometric constraint derived from a structure of lower limbs of the subject in a case where both feet of the subject are grounded based on the acquired information regarding the stride length and the angle.

ADVANTAGEOUS EFFECTS OF INVENTION

According to one aspect of the present invention, an effect can be obtained that an angle of a knee joint of a subject or an angle of a hip joint of the subject can be accurately calculated by using a small number of sensors.

BRIEF DESCRIPTION OF DRAWINGS

FIG. 1 is an explanatory diagram illustrating an example of an information processing method according to an embodiment.
FIG. 2 is an explanatory diagram illustrating an example of an information processing system 200.
FIG. 3 is a block diagram illustrating an exemplary hardware configuration of an information processing apparatus 100.
FIG. 4 is a block diagram illustrating an exemplary hardware configuration of a measuring instrument 201.
FIG. 5 is a block diagram illustrating an exemplary functional configuration of the information processing apparatus 100.
FIG. 6 is an explanatory diagram (part 1) illustrating a flow of calculating an angle of each knee joint and an angle of a hip joint.
FIG. 7 is an explanatory diagram (part 2) illustrating the flow of calculating the angle of each knee joint and the angle of the hip joint.
FIG. 8 is an explanatory diagram (part 1) illustrating an example of specifying a stable walking period.
FIG. 9 is an explanatory diagram (part 2) illustrating an example of specifying the stable walking period.
FIG. 10 is an explanatory diagram (part 3) illustrating an example of specifying the stable walking period.
FIG. 11 is an explanatory diagram (part 4) illustrating an example of specifying the stable walking period.
FIG. 12 is an explanatory diagram (part 1) illustrating an example of calculating a shin angle in a both-leg grounding period.
FIG. 13 is an explanatory diagram (part 2) illustrating an example of calculating the shin angle in the both-leg grounding period.
FIG. 14 is an explanatory diagram (part 1) illustrating a specific example in which the information processing apparatus 100 acquires gyro data, acceleration data, and position information.
FIG. 15 is an explanatory diagram (part 2) illustrating the specific example in which the information processing apparatus 100 acquires the gyro data, the acceleration data, and the position information.
FIG. 16 is an explanatory diagram (part 3) illustrating the specific example in which the information processing apparatus 100 acquires the gyro data, the acceleration data, and the position information.
FIG. 17 is an explanatory diagram (part 4) illustrating the specific example in which the information processing apparatus 100 acquires the gyro data, the acceleration data, and the position information.
FIG. 18 is an explanatory diagram (part 5) illustrating the specific example in which the information processing apparatus 100 acquires the gyro data, the acceleration data, and the position information.
FIG. 19 is an explanatory diagram (part 6) illustrating the specific example in which the information processing apparatus 100 acquires the gyro data, the acceleration data, and the position information.
FIG. 20 is an explanatory diagram (part 7) illustrating the specific example in which the information processing apparatus 100 acquires the gyro data, the acceleration data, and the position information.
FIG. 21 is an explanatory diagram (part 1) illustrating a specific example in which the information processing apparatus 100 specifies the stable walking period.
FIG. 22 is an explanatory diagram (part 2) illustrating the specific example in which the information processing apparatus 100 specifies the stable walking period.
FIG. 23 is an explanatory diagram (part 3) illustrating the specific example in which the information processing apparatus 100 specifies the stable walking period.
FIG. 24 is an explanatory diagram (part 4) illustrating the specific example in which the information processing apparatus 100 specifies the stable walking period.
FIG. 25 is an explanatory diagram (part 5) illustrating the specific example in which the information processing apparatus 100 specifies the stable walking period.
FIG. 26 is an explanatory diagram (part 6) illustrating the specific example in which the information processing apparatus 100 specifies the stable walking period.

FIG. 27 is an explanatory diagram (part 1) illustrating a specific example in which the information processing apparatus 100 specifies the both-leg grounding period.

FIG. 28 is an explanatory diagram (part 2) illustrating the specific example in which the information processing apparatus 100 specifies the both-leg grounding period.

FIG. 29 is an explanatory diagram (part 3) illustrating the specific example in which the information processing apparatus 100 specifies the both-leg grounding period.

FIG. 30 is an explanatory diagram (part 1) illustrating a specific example in which the information processing apparatus 100 calculates the shin angle.

FIG. 31 is an explanatory diagram (part 2) of the specific example in which the information processing apparatus 100 calculates the shin angle.

FIG. 32 is an explanatory diagram (part 3) of the specific example in which the information processing apparatus 100 calculates the shin angle.

FIG. 33 is an explanatory diagram (part 4) of the specific example in which the information processing apparatus 100 calculates the shin angle.

FIG. 34 is an explanatory diagram illustrating a specific example in which the information processing apparatus 100 calculates a stride length.

FIG. 35 is an explanatory diagram (part 1) illustrating a specific example in which the information processing apparatus 100 calculates each knee joint angle and a hip joint angle.

FIG. 36 is an explanatory diagram (part 2) illustrating the specific example in which the information processing apparatus 100 calculates each knee joint angle and the hip joint angle.

FIG. 37 is an explanatory diagram illustrating an exemplary output.

FIG. 38 is an explanatory diagram illustrating a specific example in which the information processing apparatus 100 uses a calculation result.

FIG. 39 (39A, 39B) is a flowchart illustrating an exemplary angle calculation processing procedure.

DESCRIPTION OF EMBODIMENTS

Hereinafter, embodiments of an information processing apparatus, an information processing system, and an information processing method according to the present invention will be described in detail with reference to the drawings.

(One Example of Information Processing Method According to Embodiment)

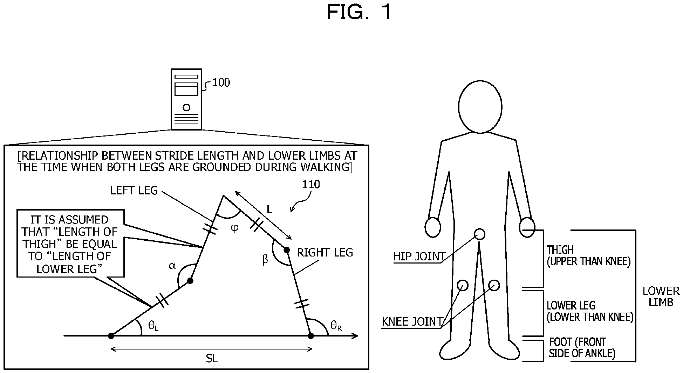

FIG. 1 is an explanatory diagram illustrating an example of an information processing method according to an embodiment. An information processing apparatus 100 is a computer that calculates at least one of an angle of a knee joint of a subject and an angle of a hip joint of the subject. The subject is a living body. The subject is, for example, a human.

For example, the subject is a person to be examined by a medical institution. Specifically, the subject is a patient who receives diagnosis, medical care, follow-up, health management, or the like by a medical person such as a medical doctor. Specifically, the subject may be a patient who receives a rehabilitation instruction or the like from a medical person such as a medical doctor. Alternatively, the subject may specifically be a trainee who is given an exercise instruction by a sports instructor. The subject may be, for example, an individual who manages his/her own health. The subject may be an animal.

Here, as described above, in a medical field, it is required to grasp a condition of the patient in order to perform an appropriate medical treatment for the patient. For example, a walking form of the patient tends to be influenced by the degree of physical disorder due to a disease, the degree of recovery of a disease or injury, and the like, and there is a case where it is required to grasp the walking form of the patient in order to grasp the condition of the patient. For example, it is required to grasp whether or not the patient walks in an unnatural walking form, like without bending both legs, that applies a load to the patient's body and may cause an accident such as falling or a fall.

Therefore, as an objective index of the patient's walking form, it is required to accurately calculate an angle of a knee joint of the patient and an angle of a hip joint of the patient and to enable to grasp the patient's walking form based on the angle of the knee joint of the patient and the angle of the hip joint of the patient. In particular, to enable to grasp the daily patient's walking form and grasp the patient's walking form outside the medical institution is preferable for applying appropriate medical care for the patient.

However, there is a case where it is difficult to accurately measure the angle of the knee joint of the patient, the angle of the hip joint of the patient, and the like. For example, if the sensor devices are not attached to various parts of the lower limbs of the patient, there is a case where it is difficult to measure the angle of the knee joint of the patient and the angle of the hip joint of the patient. Specifically, if the sensor devices are not attached to six parts including thighs, lower legs, and feet of both legs of the patient, there is a case where it is difficult to measure the angle of the knee joint of the patient and the angle of the hip joint of the patient.

On the other hand, when the sensor devices are attached to various parts of the lower limbs of the patient in order to improve accuracy of measuring the angle of the knee joint of the patient and the angle of the hip joint of the patient, this increases a load of the patient. For example, when wearing the sensor devices on the thighs, the patient tends to feel difficulty in walking. Then, there is a possibility that the patient feels the load and stress and reduces willingness to use the sensor devices so as to receive diagnosis, medical care, follow-up, health management, or the like, for the patient.

Therefore, in order to measure the angle of the knee joint of the patient and the angle of the hip joint of the patient on a daily basis, it is preferable that the number of sensor devices to be worn by the patient be relatively small and the part where the sensor device is attached be a part where the load and the stress felt by the patient are relatively small. Specifically, it is preferable that the sensor devices be attached to only the lower legs of the patient without attaching the sensor devices to the thighs of the patient.

Therefore, in the present embodiment, an information processing method will be described that can accurately measure the angle of the knee joint of the patient and the angle of the hip joint of the patient, even in a case where the sensor devices are attached to only the lower legs of the patient, by considering the geometric constraint derived from the structure of the lower limbs of the patient.

As illustrated in FIG. 1, there is a case where a portion that is a part of the leg of the subject, is a portion upper than the knee of the subject, and is a portion from the knee to the hip of the subject is referred to as "thigh". Furthermore, there is a case where the thigh of the left leg is referred to as "left thigh". Furthermore, there is a case where the thigh of the right leg is referred to as "right thigh".

Furthermore, as illustrated in FIG. 1, there is a case where a portion that is a part of the leg of the subject, is a portion lower than the knee of the subject, and is a portion from the knee to the ankle of the subject is referred to as "lower leg". Furthermore, there is a case where the lower leg of the left leg is referred to as "left lower leg". Furthermore, there is a case where the lower leg of the right leg is referred to as "right lower leg".

Furthermore, as illustrated in FIG. 1, there is a case where a portion that is a part of the leg of the subject and a portion on the front side from the ankle is referred to as "foot". Furthermore, there is a case where the foot of the left leg is referred to as "left foot". Furthermore, there is a case where the foot of the right leg is referred to as "right foot". In the example in FIG. 1, for example, the subject wears a sensor device on the lower leg.

(1-1) The information processing apparatus 100 acquires information regarding a stride length SL of the subject and information regarding angles $\theta_L$ and $\theta_R$ of the respective lower legs of the with respect to the ground in a case where both feet of the subject are grounded. Here, each of the angles $\theta_L$ and $\theta_R$ of the respective lower legs with respect to the ground is an angle of the front side of each lower leg with respect to the ground on a sagittal plane. The ground is an indoor floor, an outdoor ground surface, an outdoor road, and the like.

The information processing apparatus 100 calculates the stride length SL of the subject, for example, based on a movement distance and the number of steps of the subject. For example, the information processing apparatus 100 may acquire information regarding the stride length SL of the subject that is set in advance. For example, by integrating an angular velocity measured by the sensor device attached to each lower leg, the information processing apparatus 100 calculates the angles $\theta_L$ and $\theta_R$ of the respective lower legs with respect to the ground. With this calculation, the information processing apparatus 100 can acquire each piece of information used when angles $\alpha$ and $\beta$ of the respective knee joints of the subject and an angle $\varphi$ of the hip joint of the subject are calculated.

(1-2) The information processing apparatus 100 calculates the angles $\alpha$ and $\beta$ of the respective knee joints of the subject and the angle $\varphi$ of the hip joint of the subject that satisfy geometric constraints, derived from a structure of lower limbs of the subject in a case where both feet of the subject are grounded, based on the information regarding the stride length SL and the information regarding the angles $\theta_L$ and $\theta_R$. Here, each of the angles $\alpha$ and $\beta$ of the respective knee joints is an angle indicating a bending degree of each knee on the sagittal plane, and is an angle of an inner side of the knee. The angle $\varphi$ of the hip joint is an angle indicating an opening degree of both legs on the sagittal plane.

As illustrated in FIG. 1, the structure of the lower limbs of the subject is a structure in which the left thigh and the right thigh are connected by the hip joint, the left thigh and the left lower leg are connected by a left knee joint, and the right thigh and the right lower leg are connected by a right knee joint, and the lengths of the thigh and the lower leg are approximated to the same length L. In a case where this approximation is not satisfied, in other words, in a case where the length of the thigh is different from the length of the lower leg, the length of the thigh is indicated as L1, and the length of the lower leg is indicated as L2. A simultaneous equation to be described later may be derived based on first and second constraints to be described later.

The geometric constraint is a constraint that is geometrically derived based on a polygon 110 that is formed by using each thigh, each lower leg, and the ground as sides and the hip joint, each knee joint, and a contact point between each lower leg and the ground as vertices, by using the structure of the lower limbs of the subject. For example, the first and second constraints can be derived based on the polygon 110.

The first constraint is a constraint indicating a relationship between the stride length SL of the subject, the length L of the thigh of the subject, the length L of the lower leg of the subject, the angles $\alpha$ and $\beta$ of the respective knee joints of the subject, and the angle $\varphi$ of the hip joint of the subject in the polygon 110. The stride length SL of the subject corresponds to a length of the ground that is one side of the polygon 110.

The second constraint is a constraint indicating a relationship in which heights of the waist of the subject that can be calculated from this polygon 110 by using two calculation methods coincide with each other. The two calculation methods include, for example, a calculation method using information regarding the left leg and a calculation method using information regarding the right leg.

The calculation method using the information regarding the right leg is a calculation method, for example, based on the length L of the thigh of the subject, the length L of the lower leg of the subject, the angle $\alpha$ of the right knee joint of the subject, and the angle $\varphi$ of the hip joint of the subject. The calculation method using the information regarding the left leg is a calculation method, for example, based on the length L of the thigh of the subject, the length L of the lower leg of the subject, the angle $\beta$ of the left knee joint of the subject, and the angle $\varphi$ of the hip joint of the subject.

The information processing apparatus 100 generates, for example, a simultaneous equation including an expression indicating the first constraint and an expression indicating the second constraint based on the stride length SL and the angles $\theta_L$ and $\theta_R$ and calculates the angles $\alpha$ and $\beta$ of the respective knee joints of the subject and the angle $\varphi$ of the hip joint of the subject by solving the simultaneous equation.

As a result, the information processing apparatus 100 can accurately calculate the angles $\alpha$ and $\beta$ of the respective knee joints of the subject and the angle $\varphi$ of the hip joint of the subject even if the sensor devices are not attached to various parts of the lower limbs of the subject. For example, when the subject wears the sensor devices on the lower legs of the subject where a load and stress felt by the subject in a case where the subject wears the sensor device are relatively small, the information processing apparatus 100 can accurately calculate the angles $\alpha$ and $\beta$ of the respective knee joints of the subject and the angle $\varphi$ of the hip joint of the subject. Then, the information processing apparatus 100 can obtain an objective index regarding the walking form of the subject.

Since it is sufficient for the subject to wear a small number of sensor devices and to wear the sensor device at the part where the load and the stress felt by the subject are relatively small, the subject is less likely to feel difficulty in walking and to feel the load and stress. Then, the subject can suppress reduction in willingness to use the sensor devices so as to receive diagnosis, medical care, follow-up, health management, or the like. As a result, the subject can easily use the sensor device on a daily basis, and the information processing apparatus 100 can calculate the usual angles $\alpha$ and $\beta$ of the respective knee joints of the subject and the usual angle $\varphi$ of the hip joint of the subject.

Furthermore, if the information processing apparatus 100 outputs the angles $\alpha$ and $\beta$ of the respective knee joints of the subject and the angle $\varphi$ of the hip joint of the subject, the information processing apparatus 100 can notify a medical person such as a medical doctor of the angles $\alpha$ and $\beta$ of the respective knee joints of the subject and the angle $\varphi$ of the hip joint of the subject. Therefore, the information processing apparatus 100 can make it easier for a medical person such as a medical doctor to grasp the walking form of the subject, and can allow the medical person to efficiently perform diagnosis, medical care, follow-up, health management, or the like on the subject.

The medical person such as a medical doctor can grasp the walking form of the subject with reference to the angles $\alpha$ and $\beta$ of the respective knee joints of the subject and the angle $\varphi$ of the hip joint of the subject and efficiently perform the diagnosis, the medical care, the follow-up, the health management, or the like on the subject. The medical person such as a medical doctor can grasp the usual walking form of the subject, for example, with reference to the usual angles $\alpha$ and $\beta$ of the respective knee joints of the subject and the usual angle $\varphi$ of the hip joint of the subject.

Furthermore, the information processing apparatus 100 can reduce the number of sensor devices to be attached to the subject. Therefore, the information processing apparatus 100 can reduce cost caused when the sensor device is used to perform the diagnosis, the medical care, the follow-up, the health management, or the like on the subject.

Here, to accurately calculate the stride length SL of the subject based on the movement distance and the number of steps of the subject by the information processing apparatus 100, it is preferable that the movement distance and the number of steps of the subject be respectively a movement distance and the number of steps when the subject stably walks straight at a constant stride.

Similarly, to accurately calculate the angle of each lower leg with respect to the ground by integrating the angular velocity of each lower leg by the information processing apparatus 100, it is preferable that the angular velocity of each lower leg be an angular velocity when the subject stably walks straight at a constant stride.

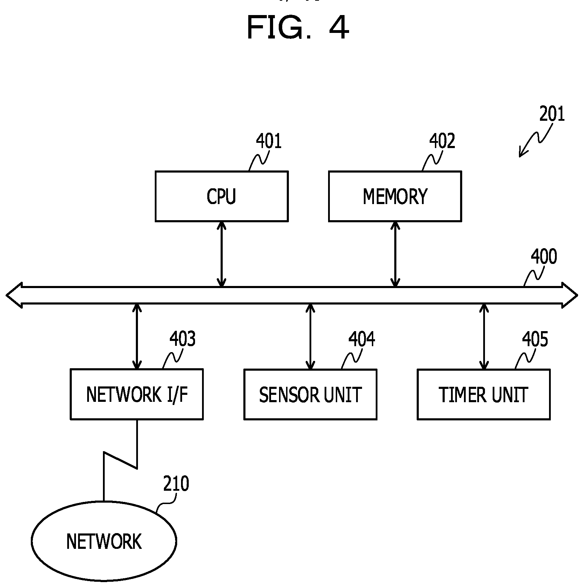

Therefore, it is preferable that the information processing apparatus 100 first specify a walking period when the subject stably walks straight at a constant stride and acquire a movement distance, the number of steps, an angular velocity of each lower leg, and the like of the subject in the walking period. A specific example in which the information processing apparatus 100 specifies the walking period when the subject stably walks straight at a constant stride will be described later with reference to FIGs. 4 to 9.

(One Example of Information Processing System 200)

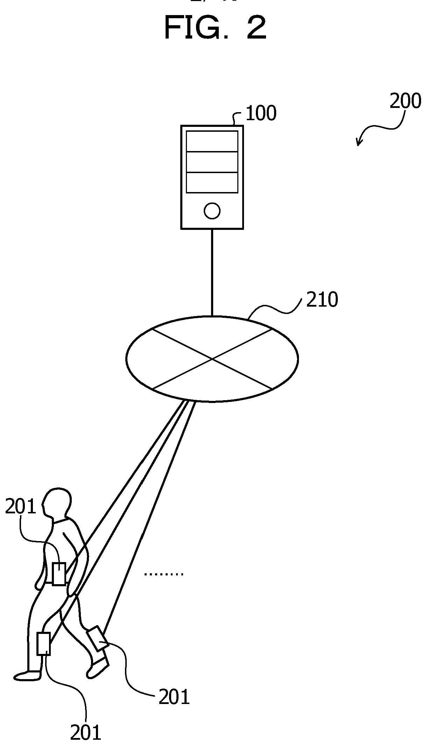

Next, an example of an information processing system 200 to which the information processing apparatus 100 illustrated in FIG. 1 will be described with reference to FIG. 2.

FIG. 2 is an explanatory diagram illustrating an example of the information processing system 200. In FIG. 2, the information processing system 200 includes the information processing apparatus 100 and one or more measuring instruments 201. In the information processing system 200, the information processing apparatus 100 and the measuring instrument 201 are connected via a wired or wireless network 210. The network 210 is, for example, a Local Area Network (LAN), a Wide Area Network (WAN), the Internet, and the like.

The information processing apparatus 100 is a computer that acquires measured information regarding a movement of each of the lower legs of the subject to be measured from the one or more measuring instruments 201. The information processing apparatus 100 calculates an angle of the lower leg of the subject with respect to the ground based on the measured information and calculates the angle of each knee joint of the subject and the angle of the hip joint of the subject. The information processing apparatus 100 includes, for example, a server, a Personal Computer (PC), a notebook PC, a tablet terminal, a smartphone, a wearable terminal, and the like.

The measuring instrument 201 is a computer attached to the lower leg of the subject to be measured. The measuring instrument 201 generates the measured information and transmits the measured information to the information processing apparatus 100. The measuring instrument 201 includes, for example, a sensor unit illustrated in FIG. 4, generates time-series data in which a measured value of the sensor unit and a measurement time at which the measured value of the sensor unit is obtained are associated with each other as the measured information, and transmits the time-series data to the information processing apparatus 100. The time-series data is, for example, sensor data indicating a waveform of the measured value.

Specifically, the measuring instrument 201 transmits sensor data regarding an angular velocity and an acceleration of the lower leg of the subject to the information processing apparatus 100. In the following description, there is a case where the sensor data regarding the angular velocity is referred to as "gyro data". There is a case where the sensor data regarding the acceleration is referred to as "acceleration data". The measuring instrument 201 is, for example, a sensor device. The sensor device is specifically a device called a motion sensor. The measuring instrument 201 may be, for example, a smartphone, a wearable terminal, and the like.

Here, a case has been described where the measuring instrument 201 transmits the sensor data regarding the angular velocity and the acceleration of the lower leg of the subject to the information processing apparatus 100. However, the present invention is not limited to this. For example, there may be a case where the measuring instrument 201 calculates the angle of the lower leg of the subject with respect to the ground based on the sensor data regarding the angular velocity and the acceleration of the lower leg of the subject and transmits the angle of the lower leg of the subject with respect to the ground to the information processing apparatus 100.

Here, a case where the measuring instrument 201 generates the sensor data has been described. However, the present invention is not limited to this. For example, the information processing apparatus 100 may sequentially receive, from the measuring instrument 201, correspondence information in which a measured value of the sensor unit of the measuring instrument 201 and a measurement time at which the measured value of the sensor unit is obtained are associated with each other, and create sensor data in which the correspondence information is collected.

Here, a case where the information processing apparatus 100 and the measuring instrument 201 are different devices has been described. However, the present invention is not limited to this. For example, the information processing apparatus 100 may be integrated with the measuring instrument 201. Here, a case where a single measuring instrument 201 is attached to the subject has been described. However, the present invention is not limited to this. For example, there may be a case where the subject wears the plurality of measuring instruments 201.

There may be a case where the information processing system 200 further includes the measuring instrument 201 that generates position information of the subject. The position information is, for example, information indicating a change in Global Positioning Systems (GPS) coordinates of the subject and the like. The measuring instrument 201 transmits the generated position information to the information processing apparatus 100.

There may a case where the information processing system 200 further includes a display device. In this case, as a result of specifying the walking period, the information processing apparatus 100 displays a predetermined feature quantity regarding the walking period, and the like via the display device. The display device is, for example, a PC, a notebook PC, a tablet terminal, a smartphone, and the like.

For example, the information processing system 200 is applied to implement a watching service for grasping a condition of a patient, or is applied to implement a service for a Personal Health Record (PHR).

(Exemplary Hardware Configuration of Information Processing Apparatus 100)

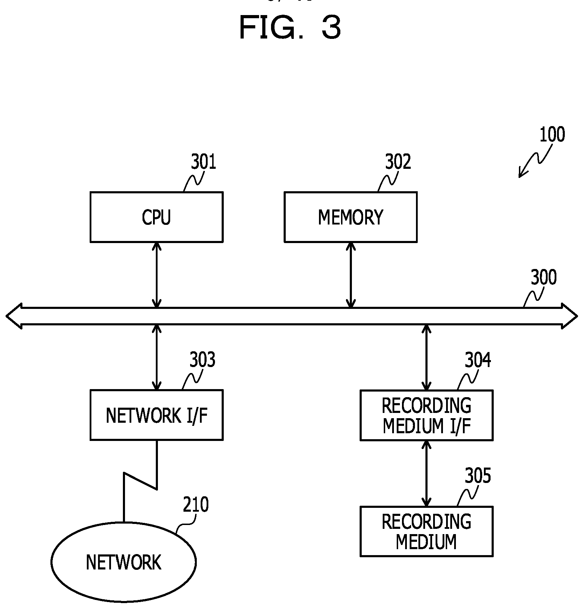

Next, an exemplary hardware configuration of the information processing apparatus 100 included in the information processing system 200 illustrated in FIG. 2 will be described with reference to FIG. 3.

FIG. 3 is a block diagram illustrating an exemplary hardware configuration of the information processing apparatus 100. In FIG. 3, the information processing apparatus 100 includes a Central Processing Unit (CPU) 301, a memory 302, a network Interface (I/F) 303, a recording medium I/F 304, and a recording medium 305. Furthermore, the components are connected by a bus 300.

Here, the CPU 301 performs overall control of the information processing apparatus 100. The memory 302 includes a Read Only Memory (ROM), a Random Access Memory (RAM), a flash ROM, and the like, for example. Specifically, for example, the flash ROM or the ROM stores various programs, while the RAM is used as a work area for the CPU 301. A program stored in the memory 302 is loaded to the CPU 301 to cause the CPU 301 to execute coded processing.

The network I/F 303 is connected to the network 210 through a communication line, and is connected to another computer through the network 210. Then, the network I/F 303 manages the network 210 and an internal interface and controls input/output of data to/from another computer. The network I/F 303 is, for example, a modem, a LAN adapter, and the like.

The recording medium I/F 304 controls read/write of data to/from the recording medium 305 under the control of the CPU 301. The recording medium I/F 304 is, for example, a disk drive, a Solid State Drive (SSD), a Universal Serial Bus (USB) port, and the like. The recording medium 305 is a nonvolatile memory that stores the data written under the control of the recording medium I/F 304. The recording medium 305 includes, for example, a disk, a semiconductor memory, a USB memory, and the like. The recording medium 305 may be removably installed on the information processing apparatus 100.

The information processing apparatus 100 may further include, for example, a keyboard, a mouse, a display, a printer, a speaker, a touch panel, or the like, in addition to the components described above. Furthermore, it is not necessary for the information processing apparatus 100 to include the recording medium I/F 304 and the recording medium 305.

(Exemplary Hardware Configuration of Measuring Instrument 201)

Next, an exemplary hardware configuration of the measuring instrument 201 included in the information processing system 200 illustrated in FIG. 2 will be described with reference to FIG. 4.

FIG. 4 is a block diagram illustrating the exemplary hardware configuration of the measuring instrument 201. In FIG. 4, the measuring instrument 201 includes a CPU 401, a memory 402, a network I/F 403, a sensor unit 404, and a timer unit 405. Furthermore, the components are connected by a bus 400.

Here, the CPU 401 performs overall control of the measuring instrument 201. The memory 402 includes, for example, a ROM, a RAM, a flash ROM, and the like. Specifically, for example, the flash ROM or the ROM stores various programs, while the RAM is used as a work area for the CPU 401. A program stored in the memory 402 is loaded to the CPU 401 to cause the CPU 401 to execute coded processing.

The network I/F 403 is connected to the network 210 through a communication line, and is connected to another computer through the network 210. In addition, the network I/F 403 manages the network 210 and an internal interface and controls input/output of data to/from another computer. The network I/F 403 is, for example, a communication circuit compliant to the Wi-Fi (registered trademark), a communication circuit compliant to the Bluetooth (registered trademark), and the like. The network I/F 403 may be, for example, a communication circuit compliant to the 3rd Generation (3G).

The sensor unit 404 measures a condition of the measuring instrument 201. The sensor unit 404 measures, for example, at least one of a position, a movement, and an orientation of the measuring instrument 201. Specifically, the sensor unit 404 includes at least one of an acceleration sensor, an angular velocity sensor, a terrestrial magnetism sensor, an optical sensor, a vibration sensor, and the like. Furthermore, the sensor unit 404 may include a GPS receiver and detect the GPS coordinates of the measuring instrument 201. The timer unit 405 measures a current time.

The measuring instrument 201 may further include, for example, a keyboard, a mouse, a display, a printer, a speaker, a touch panel, or the like, in addition to the components described above. Furthermore, the measuring instrument 201 may further include a recording medium I/F or a recording medium, in addition to the components described above. The recording medium may be removably installed on the measuring instrument 201.

(Exemplary Functional Configuration of Information Processing Apparatus 100)

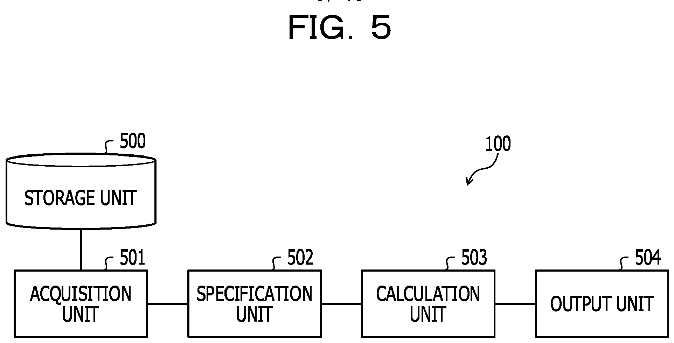

Next, an exemplary functional configuration of the information processing apparatus 100 will be described with reference to FIG. 5.

FIG. 5 is a block diagram illustrating the exemplary functional configuration of the information processing apparatus 100. The information processing apparatus 100 includes a storage unit 500, an acquisition unit 501, a specification unit 502, a calculation unit 503, and an output unit 504.

For example, the storage unit 500 is implemented by a storage area such as the memory 302 or the recording medium 305 illustrated in FIG. 3. The acquisition unit 501 to the output unit 504 are functions to be a controller. Specifically, for example, the acquisition unit 501 to the output unit 504 implement functions thereof by causing the CPU 301 to execute a program stored in a storage area such as the memory 302 or the recording medium 305 illustrated in FIG. 3 or by the network I/F 303. A processing result of each functional unit is stored in the storage area such as the memory 302 or the recording medium 305, illustrated in FIG. 3, for example.

The storage unit 500 stores measured information regarding the movement of each lower leg of the subject. The subject is, for example, a patient. The storage unit 500 stores, for example, sensor data in which the measured value of the sensor unit of the measuring instrument 201 attached to each shin of the subject is associated with the measurement time when the measured value is obtained and the values are arranged in time-series.

The measured value includes, for example, at least one of an angular velocity on a sagittal plane, an angular velocity on a traverse plane, and an angular velocity on a coronal plane. The measured value may include, for example, at least one of an acceleration in a vertical direction, an acceleration in a lateral direction, and an acceleration in a front-back direction. The measured value may include, for example, a magnitude, a position, and the like of vibration. Accordingly, the storage unit 500 can refer to the sensor data.

The storage unit 500 stores content of the geometric constraint derived from the structure of the lower limbs of the subject in a case where the both feet of the subject are grounded. The geometric constraint is a constraint that is geometrically derived based on a polygon that is formed by using each thigh, each lower leg, and the ground as sides and the hip joint, each knee joint, and a contact point between each lower leg and the ground as vertices. Here, an angle of the knee joint is an angle indicating a bending degree of each knee on the sagittal plane and is an angle of an inner side of the knee. The angle of the knee joint may be, for example, an angle of an outer side of the knee on the sagittal plane. An angle of the hip joint is an angle indicating an opening degree of both thighs on the sagittal plane and is an angle formed below the hip joint by both thighs. The angle of the hip joint may be, for example, an angle formed above the hip joint by both thighs.

The geometric constraint includes, for example, the first and second constraints derived based on the polygon. The first constraint is a constraint indicating a relationship between the stride length of the subject, the length of the thigh of the subject, the length of the lower leg of the subject, the angle of each knee joint of the subject, and the angle of the hip joint of the subject in this polygon. The stride length of the subject corresponds to a length of the ground to be one side of the polygon.

The second constraint is a constraint indicating a relationship in which heights of the waist of the subject that can be calculated from this polygon by using two calculation methods coincide with each other. The two calculation methods include, for example, a calculation method using information regarding the left leg and a calculation method using information regarding the right leg.

The calculation method using the information regarding the left leg is a calculation method, for example, based on the length of the thigh of the subject, the length of the lower leg of the subject, the angle of the left knee joint of the subject, and the angle of the hip joint of the subject. The calculation method using the information regarding the right leg is a calculation method, for example, based on the length of the thigh of the subject, the length of the lower leg of the subject, the angle of the right knee joint of the subject, and the angle of the hip joint of the subject. Accordingly, the storage unit 500 can refer to the geometric constraint.

The acquisition unit 501 acquires information regarding the stride length of the subject and information regarding the angles of each lower leg of the subject with respect to the ground in a case where both feet of the subject are grounded. For example, the acquisition unit 501 receives, for example, the information regarding the stride length of the subject and the information regarding the angle of each lower leg of the subject with respect to the ground in a case where both feet of the subject are grounded from the measuring instrument 201. With this operation, the acquisition unit 501 can acquire the information used when the angle of each knee joint of the subject and the angle of the hip joint are calculated.

Here, a case has been described where the acquisition unit 501 directly acquires the information regarding the stride length and the angles from the measuring instrument 201 without generating the information. However, the present invention is not limited to this. For example, there may be a case where the acquisition unit 501 acquires the information used to generate the information regarding the stride length and the angles and provides the information to the calculation unit 503 and the calculation unit 503 generates the information regarding the stride length and the angles.

The acquisition unit 501 acquires a movement distance of the subject and the number of steps of the subject. For example, the acquisition unit 501 receives a movement distance of the subject and the number of steps of the subject in a predetermined walking period from the measuring instrument 201 and stores the received information in the storage unit 500. The predetermined walking period is a walking period that is preset. The predetermined walking period may be a walking period specified by the specification unit 502 to be described later. Accordingly, the acquisition unit 501 can provide the movement distance and the number of steps to the specification unit 502 and the calculation unit 503.

The acquisition unit 501 acquires position information. The position information is, for example, information indicating a change in the GPS coordinates of the subject. For example, the acquisition unit 501 receives the position information from the measuring instrument 201 and stores the position information in the storage unit 500. With this operation, the acquisition unit 501 can provide the position information to the specification unit 502 and the calculation unit 503.

The acquisition unit 501 acquires an angular velocity and an acceleration of the subject. The acquisition unit 501 acquires, for example, the angular velocity and the acceleration of the subject in a predetermined candidate period from the measuring instrument 201. Specifically, the acquisition unit 501 receives gyro data and acceleration data indicating changes in the angular velocity and the acceleration of the subject in the predetermined candidate period from the measuring instrument 201 and stores the data in the storage unit 500. With this operation, the acquisition unit 501 can provide the gyro data and the acceleration data to the specification unit 502 and the calculation unit 503.

For example, the acquisition unit 501 acquires an angular velocity or an acceleration of the subject in the predetermined walking period from the measuring instrument 201. Specifically, the acquisition unit 501 receives gyro data and acceleration data indicating changes in the angular velocity and the acceleration of the subject in the predetermined walking period and stores the data in the storage unit 500. The angular velocity is, for example, an angular velocity on the sagittal plane of each lower leg of the subject. With this operation, the acquisition unit 501 can provide the gyro data and the acceleration data to the specification unit 502 and the calculation unit 503.

The acquisition unit 501 acquires an acceleration in a direction along the lower leg of the subject in the predetermined walking period from the measuring instrument 201. For example, the acquisition unit 501 acquires an acceleration along the lower leg of the subject at the time when the subject is stationary from the measuring instrument 201. The time when the subject is stationary is, for example, a start time of the predetermined walking period. Specifically, the acquisition unit 501 receives the acceleration data indicating the change in the acceleration in the direction along the lower leg of the subject in the predetermined walking period from the measuring instrument 201, stores the received data in the storage unit 500, and acquires the acceleration at the time when the subject is stationary. With this operation, the acquisition unit 501 can provide the acceleration at the time when the subject is in the stationary state to the specification unit 502 and the calculation unit 503. The stationary state is, for example, a state where the subject keeps standing.

The acquisition unit 501 acquires information indicating a moving speed and a moving direction of the subject from the measuring instrument 201. The acquisition unit 501 receives the moving speed and the moving direction of the subject from the measuring instrument 201 and stores the received information in the storage unit 500. For example, the information indicating the moving speed may be information indicating a change in the GPS coordinates of the subject at each constant interval or may be the position information. Accordingly, the acquisition unit 501 can provide the moving speed and the moving direction of the subject to the specification unit 502 and the calculation unit 503.

The specification unit 502 specifies the predetermined walking period. For example, the specification unit 502 sets a stable period, in which the moving speed and the moving direction acquired by the acquisition unit 501 are constant, as a predetermined walking period. In a case where the change in the GPS coordinates of the subject at each constant interval is constant, the specification unit 502 may determine that the moving speed is constant. With this determination, the specification unit 502 can set a stable period in which the subject stably moves as a predetermined walking period and can improve calculation accuracy of the calculation unit 503 to be described later.

The specification unit 502 specifies a plurality of motion time points, in the predetermined candidate period, when a feature corresponding to one step of the subject appears in the angular velocity or the acceleration of the subject. The feature corresponding to one step is, for example, that the angular velocity exceeds a threshold and becomes a local maximum value. Then, the specification unit 502 sets the stable period, in which the intervals of the motion time points are constant, based on the plurality of specified motion time points in the predetermined candidate period, as the predetermined walking period. With this determination, the specification unit 502 can set a stable period in which the subject stably walks as a predetermined walking period and can improve calculation accuracy of the calculation unit 503 to be described later.

The specification unit 502 may set the stable period, in which the feature quantity of the motion of the subject at the motion time point is constant, based on the plurality of specified motion time points in the predetermined candidate period, as the predetermined walking period. With this determination, the specification unit 502 can set a stable period in which the subject stably walks as a predetermined walking period and can improve calculation accuracy of the calculation unit 503 to be described later.

The specification unit 502 specifies the plurality of motion time points, in the predetermined walking period, when the feature corresponding to one step of the subject appears in the acquired angular velocity or acceleration of the subject. With this operation, the specification unit 502 can provide the plurality of motion time points to the calculation unit 503.

The calculation unit 503 calculates the stride length of the subject by dividing the acquired movement distance by the number of steps. For example, the calculation unit 503 calculates the movement distance of the subject in the predetermined walking period based on the position information. Furthermore, for example, the calculation unit 503 calculates the number of times when the feature corresponding to one step of the subject appears in the gyro data in the predetermined walking period as the number of steps of the subject. Then, the calculation unit 503 calculates the stride length of the subject by dividing the movement distance by the number of steps.

The calculation unit 503 calculates the angle of each lower leg of the subject with respect to the ground at a grounding time when both feet of the subject are grounded. The calculation unit 503, for example, adds an integral value of the angular velocity acquired before the grounding time when both feet of the subject are grounded to the angle of each lower leg of the subject with respect to the ground at the time when the subject is stationary so as to calculate the angle of each lower leg of the subject with respect to the ground at the grounding time. The time when the subject is stationary is, for example, a start time of the predetermined walking period. Accordingly, the calculation unit 503 can calculate information used to calculate the angle of each knee joint of the subject and the angle of the hip joint of the subject.

The calculation unit 503 calculates the angle of each lower leg of the subject with respect to the ground at the time when the subject is stationary based on the acquired acceleration in the direction along the lower leg of the subject and the acceleration of gravity. With this calculation, the calculation unit 503 can improve the accuracy of calculating the angle of each lower leg of the subject with respect to the ground at the grounding time.

The calculation unit 503 adds an integral value of the angular velocity acquired before a first motion time point of the plurality of motion time points to the angle of each lower leg of the subject with respect to the ground at the time when the subject is stationary so as to calculate an angle of each lower leg of the subject with respect to the ground at the first motion time point. With this calculation, the calculation unit 503 can calculate the angle of each lower leg of the subject with respect to the ground at the first motion time point with relatively high accuracy.

The calculation unit 503 adds an integral value of the angular velocity acquired from a motion time point that is closest and immediately before the grounding time to the grounding time to a predetermined angle of each lower leg of the subject with respect to the ground at the first motion time point so as to calculate the angle of each lower leg of the subject with respect to the ground at the grounding time. With this calculation, the calculation unit 503 can improve the accuracy of calculating the angle of each lower leg of the subject with respect to the ground at the grounding time.

The calculation unit 503 specifies an error relative to the angular velocity of the sagittal plane of each lower leg of the subject in the predetermined walking period so that the integral value of the angular velocity of the sagittal plane of each lower leg of the subject before a final time point of the predetermined walking period becomes zero. The calculation unit 503 calculates the angle of each lower leg of the subject with respect to the ground at the grounding time based on the specified error. With this calculation, the calculation unit 503 can improve the accuracy of calculating the angle of each lower leg of the subject with respect to the ground at the grounding time.

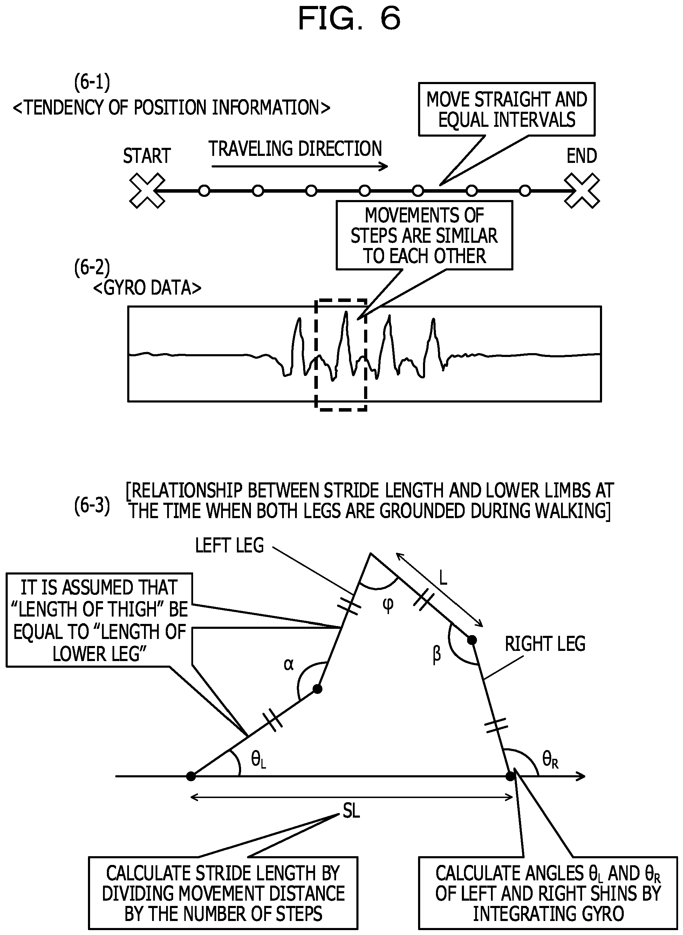

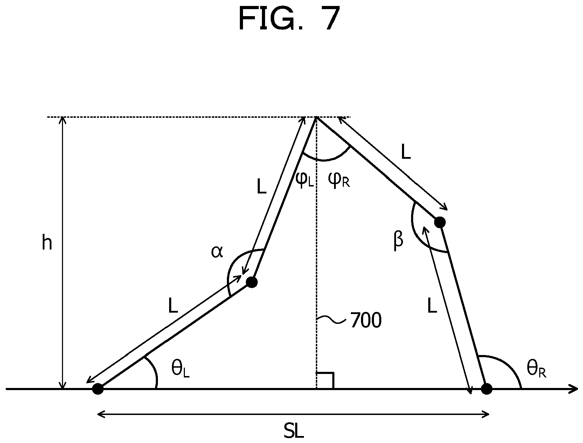

The calculation unit 503 calculates at least one of the angle of each knee joint of the subject or the angle of the hip joint of the subject that satisfies the geometric constraint stored in the storage unit 500, based on the information regarding the stride length and the angles. For example, as described later with reference to FIGs. 6 and 7, the calculation unit 503 calculates the angle of each knee joint of the subject and the angle of the hip joint of the subject. With this calculation, the calculation unit 503 can improve the accuracy of calculating the angle of each knee joint of the subject and the angle of the hip joint of the subject.

The output unit 504 outputs at least one of the angle of each knee joint of the subject or the angle of the hip joint of the subject calculated by the calculation unit 503. The output is implemented as display on a display, print output to a printer, transmission to an external device by the network I/F 303, or storage to the storage area such as the memory 302 and the recording medium 305, for example.

(Flow of Calculating Angle of Each Knee Joint And Angle of Hip Joint)

Next, a flow of calculating the angle of each knee joint and the angle of the hip joint by the information processing apparatus 100 will be described with reference to FIGs. 6 and 7. First, description of FIG. 6 will be made.

FIGs. 6 and 7 are explanatory diagrams illustrating a flow of calculating the angle of each knee joint and the angle of the hip joint. In FIG. 6, the information processing apparatus 100 specifies a walking period in which the stride length is determined to be constant.

(6-1) The information processing apparatus 100 receives, for example, the position information indicating the change in the GPS coordinates of the subject and the like from the measuring instrument 201 worn by the subject. The position information indicates, for example, GPS coordinates for each of observation points at constant intervals. Based on the received position information, the information processing apparatus 100 specifies a period in which the subject moves straight and the position of the subject changes at equal intervals.

(6-2) Based on the gyro data of the lower leg of the subject, the information processing apparatus 100 specifies a period in which it is determined that movements of each leg of the subject for the respective steps are similar and that the subject stably walks in the specified period, as the stable walking period.

Here, one step is a series of motions in which the subject separates the leg from the ground, swings the leg, and lands the leg. Here, the landing of the leg specifically indicates landing of the foot which is a part of the leg. In the following description, "landing of the leg" indicates "landing of the foot". In the following description, there is a case where one step is referred to as "step", and to separate the leg from the ground is referred to as "toe-off", and the landing of the leg is referred to as "heel-strike".

(6-3) The information processing apparatus 100 calculates the movement distance of the subject in the stable walking period based on the position information, calculates the number of steps in the stable walking period, and divides the movement distance by the number of steps so as to calculate an estimated stride length SL for each step.

Furthermore, the information processing apparatus 100 specifies a time point when both legs of the subject are grounded in the stable walking period based on the gyro data of the lower leg of the subject as a both-leg grounding time. The information processing apparatus 100 calculates the angles $\theta_L$ and $\theta_R$ of the respective lower legs with respect to the ground at the specified both-leg grounding time based on the gyro data of the lower legs of the subject. In the following description, there is a case where the angle of the lower leg with respect to the ground is referred to as a "shin angle".

Next, the information processing apparatus 100 calculates the angles $\alpha$ and $\beta$ of the respective knee joints and the angle $\varphi$ of the hip joint that satisfy a predetermined geometric constraint based on the calculated estimated stride length SL and each of the shin angles $\theta_L$ and $\theta_R$ at the specified both-leg grounding time. In the following description, there is a case where the angle of the knee joint is referred to as "knee joint angle" and the angle of the hip joint is referred to as "hip joint angle". Here, the description of FIG. 7 will be made. An example is described in which the information processing apparatus 100 calculates the angles $\alpha$ and $\beta$ of the respective knee joints and the angle $\varphi$ of the hip joint.

In FIG. 7, the information processing apparatus 100 calculates the hip joint angle $\varphi$ based on the first constraint regarding the stride length and the second constraint regarding a waist height h. Here, the hip joint angle $\varphi$ is divided into a hip joint angle $\varphi_L$ and a hip joint angle $\varphi_R$ by a perpendicular 700 from the hip joint to the ground, and the following description will be made.

The information processing apparatus 100 uses the following formula (1) indicating the first constraint using the hip joint angle $\varphi_L$ and the hip joint angle $\varphi_R$ and the following formula (2) indicating the second constraint using the hip joint angle $\varphi_L$ and the hip joint angle $\varphi_R$ as a simultaneous equation and calculates the hip joint angle $\varphi$ by solving the simultaneous equation.

[Expression 1]

$$SL = L\cos\theta_L + L\sin\phi_L + L\sin\phi_R + L\cos\theta_R \quad \ldots(1)$$

The reference L is the length of the thigh and the lower leg of the subject. For example, L is measured and set by a user of the information processing apparatus 100 in advance. For example, L may be calculated by the information processing apparatus 100 from the height of the subject that is measured in advance by the user of the information processing apparatus 100.

[Expression 2]

$$h = L\sin\theta_L + L\cos\phi_L = L\sin\theta_R + L\cos\phi_R \quad \ldots(2)$$

The reference L is the length of the thigh and the lower leg of the subject. The reference h is the height of the waist. It is not necessary to set a specific value for h.

Next, the information processing apparatus 100 calculates the knee joint angles $\alpha$ and $\beta$ based on the hip joint angle $\varphi_L$ and the hip joint angle $\varphi_R$. The information processing apparatus 100, for example, calculates the knee joint angles $\alpha$ and $\beta$ by using the following formula (3) regarding the knee joint angle $\alpha$ and the following formula (4) regarding the knee joint angle $\beta$.

[Expression 3]

$$\alpha = \frac{\pi}{2} + \theta_L + \phi_L \quad \ldots(3)$$

[Expression 4]

$$\beta = \frac{\pi}{2} + \theta_R - \phi_R \quad \dots (4)$$

The information processing apparatus 100 calculates the hip joint angle $\phi$ based on the hip joint angle $\phi_L$ and the hip joint angle $\phi_R$. With this calculation, the information processing apparatus 100 can calculate the knee joint angles $\alpha$ and $\beta$ and the hip joint angle $\phi$. Then, since it is not necessary for the information processing apparatus 100 to directly calculate the angle other than the shin angle from the gyro data, an increase in the number of measuring instruments 201 attached to the patient can be reduced.

(One Example of Calculating Angle of Each Knee Joint And Angle of Hip Joint)

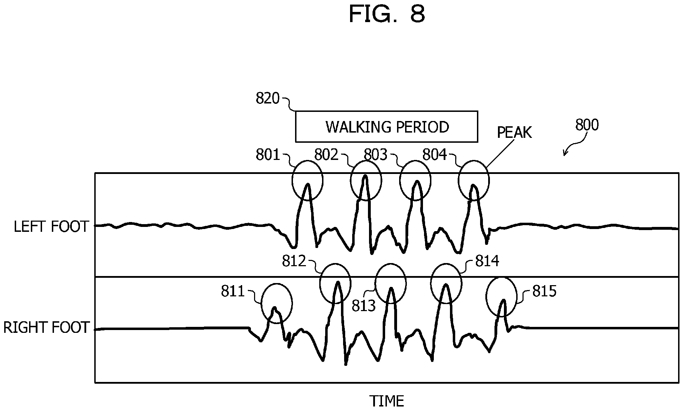

Next, an example of calculating the angle of each knee joint and the angle of the hip joint by the information processing apparatus 100 will be described with reference to FIGs. 8 to 13. First, the description of FIGs. 8 to 11 will be made. An example will be described in which the information processing apparatus 100 specifies the stable walking period.

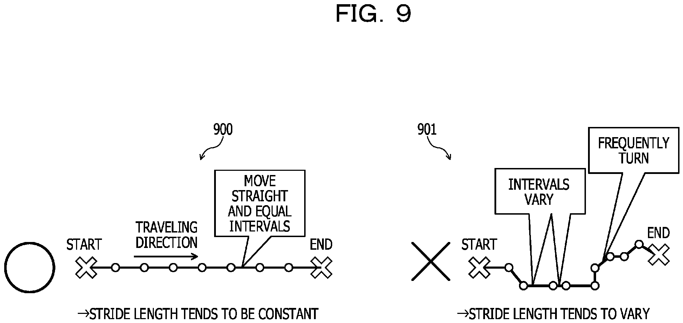

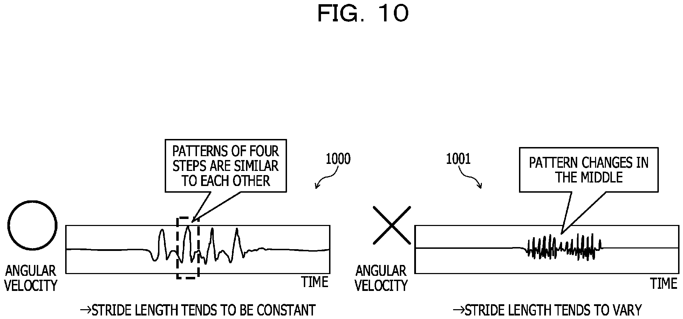

FIGs. 8 to 11 are explanatory diagrams illustrating an example of specifying the stable walking period. Specifically, the information processing apparatus 100 specifies a period in which the subject walks and moves in FIG. 8, determines whether or not the subject stably moves in the period in FIG. 9, and determines whether or not the subject stably walks in the period in FIG. 10 so as to specify the stable walking period.

In FIG. 8, first, the information processing apparatus 100 specifies a period in which the subject walks and moves equal to or more than a certain distance. In FIG. 8, it is not necessary for the information processing apparatus 100 to evaluate whether or not the period to be specified is a period in which the subject stably walks straight.

A graph 800 in FIG. 8 illustrates the gyro data of each lower leg. The horizontal axis indicates time, and the vertical axis indicates an angular velocity. The information processing apparatus 100 specifies a time point at which the angular velocity exceeds a threshold and becomes a local maximum value in the gyro data of each lower leg as a time point when the subject steps. In the following description, there is a case where a time point when the angular velocity becomes the local maximum value is referred to as "peak point".

The information processing apparatus 100 specifies, for example, peak points 801 to 804 regarding the left lower leg and peak points 811 to 815 regarding the right lower leg. Then, in a case where the peak points 801 to 804 and the peak points 811 to 815 alternately appear, the information processing apparatus 100 specifies a period 820 in which the peak points 801 to 804 and 811 to 815 appear.

The information processing apparatus 100 determines whether or not the subject moves equal to or more than a certain distance in the specified period 820 based on the position information indicating the GPS coordinates of the subject for each of the observation points at constant intervals. When the subject does not move equal to or more than a certain distance, the information processing apparatus 100 determines that the period 820 is not the stable walking period. Here, it is assumed that the information processing apparatus 100 determine that the subject moves equal to or more than a certain distance. Next, description of FIG. 9 will be made.

In FIG. 9, the information processing apparatus 100 specifies the GPS coordinates of the subject for each observation point in the period 820 based on the position information and determines whether or not the period 820 is a period in which the subject moves straight and the position of the subject tends to change at equal intervals.

The period in which the subject moves straight and the position of the subject tends to change at equal intervals is, for example, a period in which a route formed by the GPS coordinates of the subject for each observation point is provided like a route 900. Furthermore, the period in which the subject does not move straight or the position of the subject does not tend to change at equal intervals is, for example, a period in which the route formed by the GPS coordinates of the subject for each observation point is provided like a route 901.

When the route formed by the specified GPS coordinates is provided like the route 900, the information processing apparatus 100 determines that the subject stably moves. On the other hand, when the route formed by the specified GPS coordinates is provided like the route 901, the information processing apparatus 100 determines that the subject does not stably move and that the period 820 is not the stable walking period.

The information processing apparatus 100 specifically calculates a variation $E_1$ in the intervals of the observation points by using the following formula (5). Then, when the variation $E_1$ is equal to or less than a threshold, the information processing apparatus 100 determines that the position of the subject tends to change at the equal intervals.
[Expression 5]

$$E_1 = \operatorname*{var}_{1 < n < N} (|d_n|) \quad \dots (5)$$

Furthermore, the information processing apparatus 100 specifically calculates an angle $E_2$ formed by a travel vector $d_n$ from an observation point to a next observation point by using the following formula (6). Then, when the angle $E_2$ is equal to or less than a threshold, the information processing apparatus 100 determines that the subject tends to move straight.

[Expression 6]

$$E_2 = \frac{1}{N}\left|\sum_{n=1}^{N}\theta_n\right| = \frac{1}{N}\left|\sum_{n=1}^{N}arccos\left(\frac{d_{n+1}\cdot d_n}{|d_{n+1}|\times|d_n|}\right)\right| \quad ...(6)$$

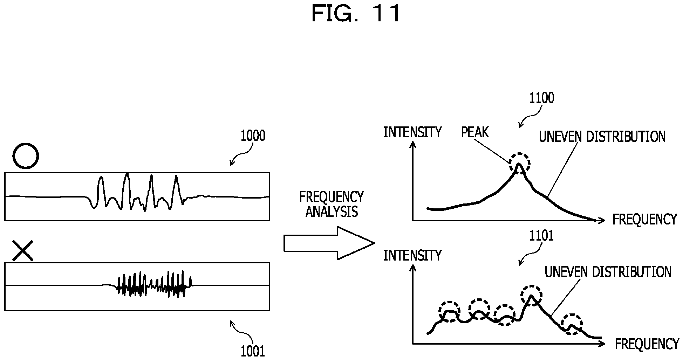

Here, it is assumed that the information processing apparatus 100 determine that the period 820 is a period in which the subject moves straight and the position of the subject tends to change at equal intervals. Next, description of FIGs. 10 and 11 will be made.

In FIGs. 10 and 11, the information processing apparatus 100 determines whether or not the period 820 is a period in which the movements of the respective legs of the subject for each step tend to be similar to each other, based on the gyro data of each lower leg.

As illustrated in FIG. 10, the period in which the movements of the respective legs of the subject for each step tend to be similar to each other is a period in which waveforms corresponding to the respective steps are similar to each other and it is determined that the stride length is constant, for example, as a period 1000. On the other hand, a period in which the movements of the respective legs of the subject for each step do not tend to be similar to each other is a period in which the waveforms are disturbed and it is determined that the stride length is not constant, for example, as a period 1001.

As illustrated in FIG. 11, when the waveforms corresponding to the respective steps are similar to each other and a frequency component in the period 1000 in which it is determined that the stride length is constant and the like is extracted, a frequency spectrum tends to have the small number of peaks as in a graph 1100. The frequency spectrum is a graph indicating a size of each frequency component. On the other hand, when a frequency component in a period 1001 in which the waveform is disturbed and it is determined that the stride length is not constant and the like is extracted, the frequency spectrum tends to have a larger number of peaks as in a graph 1101.

Therefore, the information processing apparatus 100 extracts the frequency component of the gyro data of each lower leg in the period 820. Then, the information processing apparatus 100 determines, based on the extracted frequency component, that the movements of the respective legs of the subject for each step tend to be similar to each other in the period 820 as the number of peaks of the frequency spectrum is smaller and an intensity distribution of the frequency spectrum is uneven.

Specifically, the information processing apparatus 100 calculates an evaluation value H that increases as the number of peaks of the frequency spectrum is smaller and the intensity distribution is uneven, by using the following formula (7). Then, when the evaluation value H is equal to or more than a threshold, the information processing apparatus 100 determines that the movements of the respective legs of the subject for each step tend to be similar to each other.

[Expression 7]

$$H = -\sum_{i=1}^{M} p_i \log p_i \quad ...(7)$$

The reference pi is a value that expresses a possibility by normalizing an intensity of an i-th frequency. Here, it is assumed that the information processing apparatus 100 determine that the movements of the respective legs of the subject for each step tend to be similar to each other in the period 820. As described above, since the information processing apparatus 100 determines that the subject tends to stably move and tends to stably walk in the period 820, the information processing apparatus 100 sets the period 820 as the stable walking period.

When setting the stable walking period, the information processing apparatus 100 calculates a movement distance D of the subject in the stable walking period based on the position information. Furthermore, the information processing apparatus 100 calculates the number of peak points in the stable walking period as the number of steps N in the stable walking period. Then, the information processing apparatus 100 calculates the estimated stride length SL for each step by using the following formula (8).

[Expression 8]

$$SL = \frac{D}{N} \quad \text{...} (8)$$

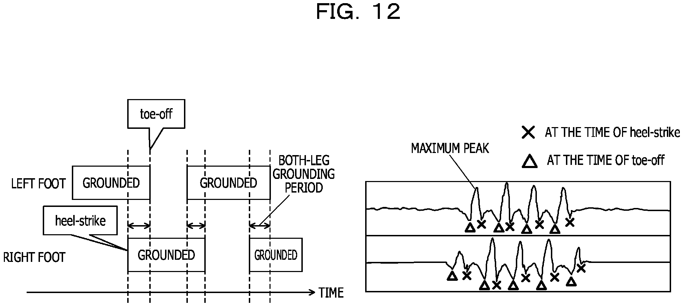

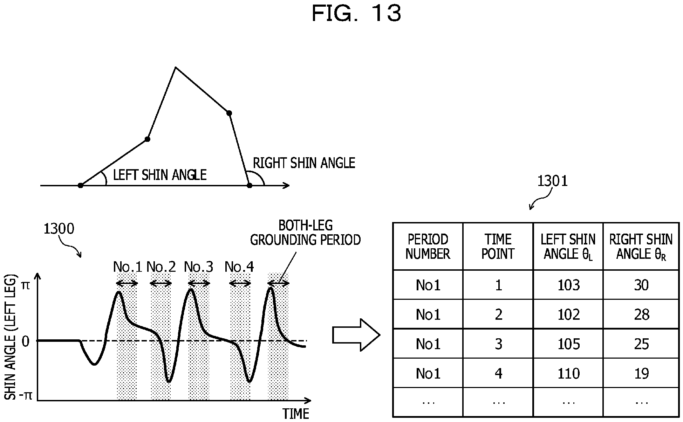

Next, description of FIGs. 12 and 13 will be made. An example will be described in which the information processing apparatus 100 calculates each shin angle in the both-leg grounding period. The both-leg grounding period is a period in which both legs of the subject are grounded.

FIGs. 12 and 13 are explanatory diagrams illustrating an example of calculating the shin angle in the both-leg grounding period. Specifically, the information processing apparatus 100 specifies the both-leg grounding period in FIG. 12 and calculates each shin angle in the both-leg grounding period in FIG. 13.

In FIG. 12, the information processing apparatus 100 specifies a support period in which one leg supports the weight of the subject for each step based on the gyro data of each lower leg.

For example, the information processing apparatus 100 specifies a time point when one leg performs heel-strike as a start point of the support period. The time point when the heel-strike is performed is a time point when the angular velocity becomes a local minimum value after becoming the local maximum value. In the following description, there is a case where the time point when the heel-strike is performed is referred to as "heel-strike point".

Furthermore, the information processing apparatus 100 specifies a time point when one leg performs toe-off as an end point of the support period. The time point when the toe-off is performed is a time point when the angular velocity becomes the local minimum value before becoming the local maximum value. In the following description, there is a case where the time point when the toe-off is performed is referred to as "toe-off point".

Then, the information processing apparatus 100 specifies a period from the heel-strike point to the toe-off point as the support period. The information processing apparatus 100 specifies a period in which different support periods overlap as the both-leg grounding period. Next, description of FIG. 13 will be made.

In FIG. 13, the information processing apparatus 100 calculates the shin angles $\theta_L$ and $\theta_R$ based on the gyro data of the respective lower legs for each specified both-leg grounding period.

For example, the information processing apparatus 100 sets each of the shin angles $\theta_L$ and $\theta_R$ at the head of the stable walking period to an initial value of zero.

Then, the information processing apparatus 100 adds the integral value of the angular velocity of each lower leg before each time point to each of the shin angles $\theta_L$ and $\theta_R$ based on the gyro data of each lower leg so as to calculate the shin angles $\theta_L$ and $\theta_R$ at each time point.

A graph 1300 in FIG. 13 illustrates the shin angle $\theta_L$ at each time point. The vertical axis indicates the shin angle $\theta_L$, and the horizontal axis indicates time. The information processing apparatus 100 can calculate the corresponding shin angle $\theta_L$ on the graph 1300 by adding the integral value of the angular velocity of each lower leg before each time point to each of the shin angles $\theta_L$ and $\theta_R$.

Then, the information processing apparatus 100 allocates a period number for each both-leg grounding period, associates the period number allocated for each both-leg grounding period, the time point when the shin angles $\theta_L$ and $\theta_R$ are calculated, and the shin angles $\theta_L$ and $\theta_R$ with each other, and stores the values by using a shin angle management table 1301.

Thereafter, the information processing apparatus 100 calculates the knee joint angles $\alpha$ and $\beta$ and the hip joint angle $\varphi$ by using the following formulas (9) to (12) based on the calculated estimated stride length SL and the calculated shin angles $\theta_L$ and $\theta_R$. Since the following formulas (9) to (12) are similar to the above formulas (1) to (4), description thereof will be omitted.

[Expression 9]

$$SL = L \cos \theta_L + L \sin \phi_L + L \sin \phi_R + L \cos \theta_R \quad \text{...} (9)$$

[Expression 10]

$$h = L \sin \theta_L + L \cos \phi_L = L \sin \theta_R + L \cos \phi_R \quad \text{...} (10)$$

[Expression 11]

$$\alpha = \frac{\pi}{2} + \theta_L + \phi_L \quad \text{...} (11)$$

[Expression 12]

$$\beta = \frac{\pi}{2} + \theta_R - \phi_R \quad \cdots (12)$$

With this calculation, the information processing apparatus 100 can calculate the knee joint angles $\alpha$ and $\beta$ and the hip joint angle $\varphi$. Then, since it is not necessary for the information processing apparatus 100 to directly calculate the angle other than the shin angle from the gyro data, an increase in the number of measuring instruments 201 attached to the patient can be reduced.

Furthermore, the information processing apparatus 100 can specify the stable walking period and calculate the knee joint angles $\alpha$ and $\beta$ and the hip joint angle $\varphi$ in the stable walking period. Therefore, the information processing apparatus 100 can prevent an adverse effect on the calculation result due to unstable walking and improve the accuracy of calculating the knee joint angles $\alpha$ and $\beta$ and the hip joint angle $\varphi$.

(Specific Example of Calculating Angle of Each Knee Joint And Angle of Hip Joint)

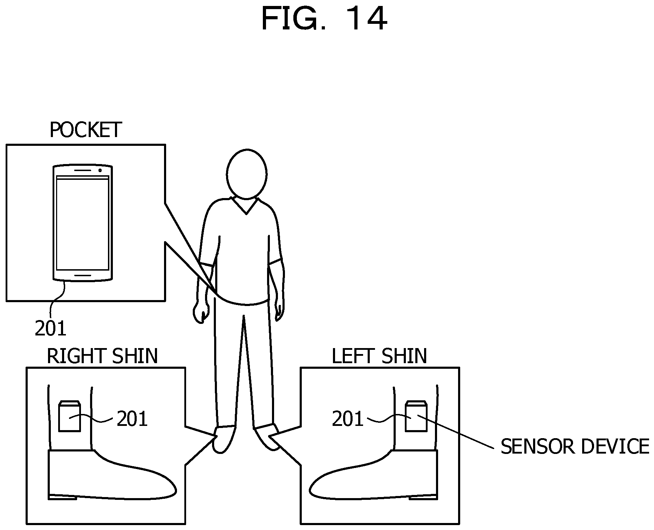

Next, a specific example of calculating the angle of each knee joint and the angle of the hip joint by the information processing apparatus 100 will be described with reference to FIGs. 14 to 38 by taking a specific data structure as an example. First, description of FIGs. 14 to 20 will be made. A specific example in which the information processing apparatus 100 acquires the gyro data, the acceleration data, and the position information will be described.

FIGs. 14 to 20 are explanatory diagrams illustrating specific examples in which the information processing apparatus 100 acquires the gyro data, the acceleration data, and the position information. In FIG. 14, the subject wears the measuring instruments 201 that measure the angular velocity and the acceleration on the right lower leg and the left lower leg. The subject wears the measuring instrument 201, for example, on the right shin or the left shin. The measuring instrument 201 worn on the right shin or the left shin measures the angular velocities and the accelerations of the right shin and the left shin and generates the gyro data and the acceleration data as sensor data in which a measured value and a measurement time are associated with each other.

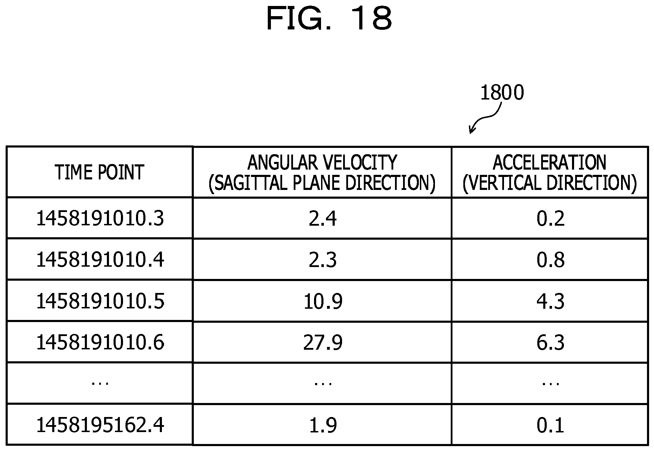

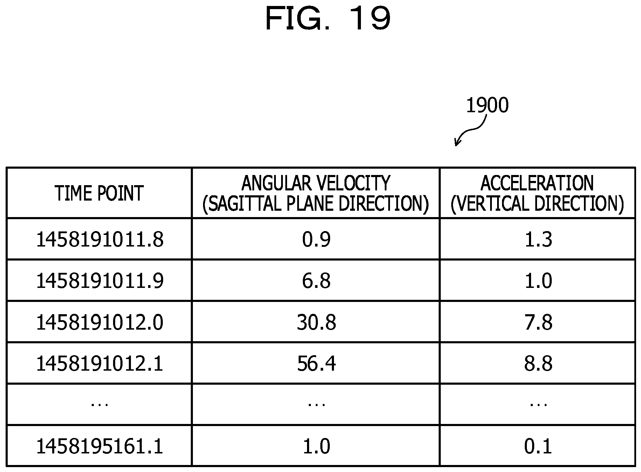

The measuring instrument 201 worn on the right shin or the left shin connects to the information processing apparatus 100 and transmits the generated gyro data and acceleration data to the information processing apparatus 100. The information processing apparatus 100 receives the gyro data, the acceleration data, and the like generated by the measuring instrument 201 worn on the right shin or the left shin. Specific examples of the gyro data and the acceleration data will be described later with reference to FIGs. 18 and 19.

Moreover, the subject wears the measuring instrument 201 that generates the position information on the waist. The subject wears the measuring instrument 201, for example, on the right shin or the left shin. The measuring instrument 201 worn on the waist measures the GPS coordinates for each of the observation points at constant intervals and generates the position information in which the measured value is associated with the measurement time.

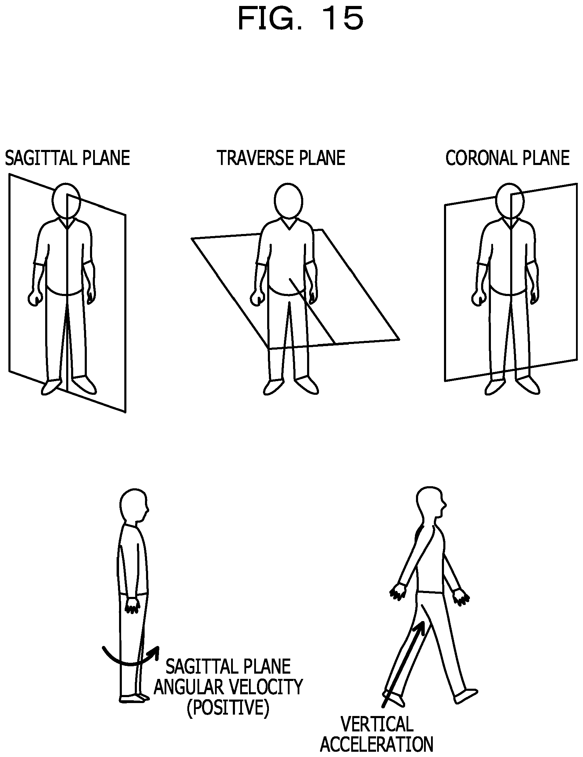

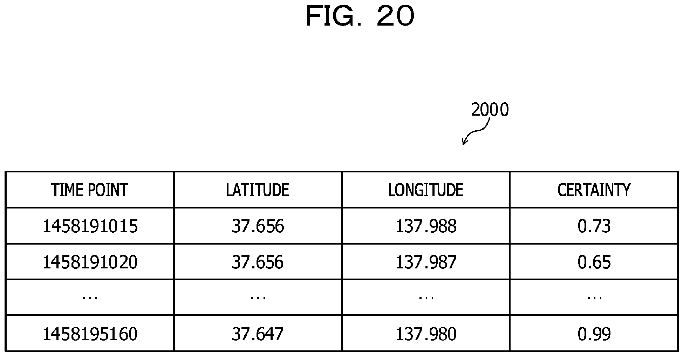

The measuring instrument 201 worn on the waist connects to the information processing apparatus 100 and transmits the generated position information to the information processing apparatus 100. The information processing apparatus 100 receives the position information generated by the measuring instrument 201 worn on the waist and the like. A specific example of the position information will be described later with reference to FIG. 20. Next, description of FIG. 15 will be made, and the contents of measurement by the measuring instruments 201 will be described.

As illustrated in FIG. 15, when measuring the angular velocity, the measuring instrument 201 measures, for example, the angular velocity on the sagittal plane, the angular velocity on the traverse plane, the angular velocity on the coronal plane, and the like. With this configuration, for example, the measuring instruments 201 generate, as sensor data, gyro data in which angular velocities on the sagittal plane are arranged in time series, gyro data in which angular velocities on the traverse plane are arranged in time series, gyro data in which angular velocities on the coronal plane are arranged in time series, and the like.

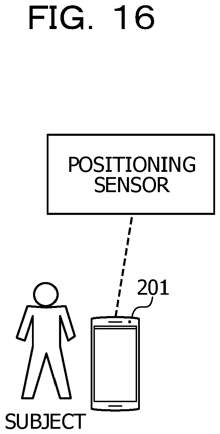

Furthermore, in a case where the acceleration is measured, for example, the measuring instrument 201 measures an acceleration in the vertical direction. Since the measuring instrument 201 is worn on the shin, the acceleration in the vertical direction may indicate the acceleration in the vertical direction along the shin of the subject. In the following description, there is a case where the acceleration in the vertical direction may be referred to as "vertical acceleration". Accordingly, for example, the measuring instrument 201 generates the acceleration data in which the vertical accelerations are arranged in time series as the sensor data. Next, description of FIG. 16 will be made. A specific example will be described in which the measuring instrument 201 generates the position information.

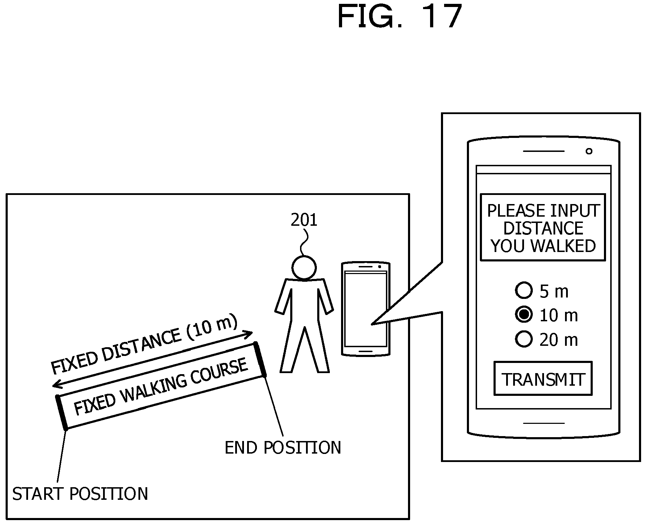

In FIG. 16, the measuring instrument 201 worn on the waist may generate the position information without using the GPS coordinates and transmit the position information to the information processing apparatus 100. For example, the measuring instrument 201 may generate the position information by communicating with a positioning sensor that measures a distance to the subject. The positioning sensor is, for example, a laser range finder the Kinect (registered trademark), or the like. Next, description of FIG. 17 will be made, and a specific example in which the measuring instrument 201 generates the position information will be described.

In FIG. 17, the measuring instrument 201 worn on the waist may generate the position information without using the GPS coordinates and transmit the position information to the information processing apparatus 100. The measuring instrument 201 may, for example, receive an input of the movement distance of the subject by an operation input by the subject and generate position information indicating the movement distance. In the example in FIG. 17, it is preferable that the subject walk on a straight and flat walking route and input the movement distance to the measuring instrument 201. Next, description of FIG. 18 will be made.

FIG. 18 illustrates a data structure of right shin data 1800 including the gyro data and the acceleration data generated by the measuring instrument 201 worn on the right shin. The right shin data 1800 accumulates records in which the time point at which the angular velocity and the acceleration are measured, the angular velocity, and the acceleration are associated with each other. Next, description of FIG. 19 will be made.

FIG. 19 illustrates a data structure of left shin data 1900 including the gyro data and the acceleration data generated by the measuring instrument 201 worn on the left shin. The left shin data 1900 accumulates records in which the time point at which the angular velocity and the acceleration are measured, the angular velocity, and the acceleration are associated with each other. Next, description of FIG. 20 will be made.

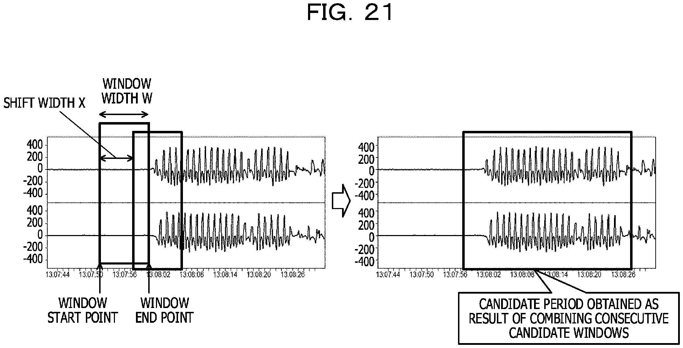

FIG. 20 illustrates a data structure of position information 2000 generated by the measuring instrument 201 worn on the waist. The position information 2000 accumulates records in which a time point when the GPS coordinates are measured, the latitude of the GPS coordinates, the longitude of the GPS coordinates, and certainty indicating accuracy of the GPS are associated with each other. Next, description of FIGs. 21 to 26 will be made, and a specific example in which the information processing apparatus 100 specifies the stable walking period will be described.

FIGs. 21 to 26 are explanatory diagram illustrating a specific example in which the information processing apparatus 100 specifies the stable walking period. In FIG. 21, the information processing apparatus 100 specifies a candidate period of a walking period in which the subject walks and moves. For example, while shifting a window having a window width W by a shift width X, the information processing apparatus 100 calculates each of motion amounts $V_{right}$ and $V_{left}$ of the respective shins by using the following formula (13) based on the gyro data of each shin corresponding to the window. In a case where each of the motion amounts $V_{right}$ and $V_{left}$ of the respective shins exceeds a threshold, the information processing apparatus 100 sets a period indicated by the window as the candidate period.

[Expression 13]

$$V_{right} = \operatorname*{mean}_{t \in W} \left| gyro_x^{(right)}(t) \right|^2, \ V_{left} = \operatorname*{mean}_{t \in W} \left| gyro_x^{(left)}(t) \right|^2 \quad \dots (13)$$

Here, the expression $gyro_x^{(right)}(t)$ is an acceleration of the sagittal plane of the right shin at a time point t. The expression $gyro_x^{(left)}(t)$ is an acceleration of the sagittal plane of the left shin at the time point t.

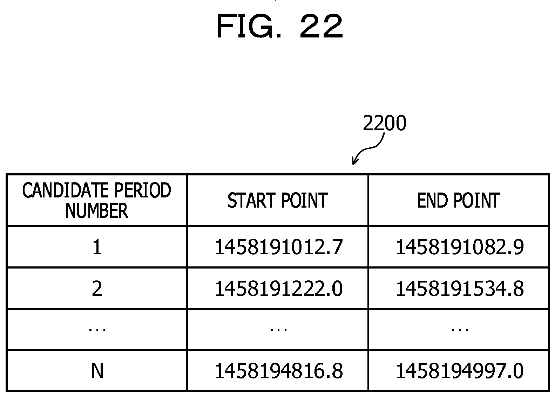

In a case where the windows set as the candidate periods are consecutive, the information processing apparatus 100 collectively sets a period indicated by the plurality of consecutive windows as a single candidate period. Next, description of FIG. 22 will be made.

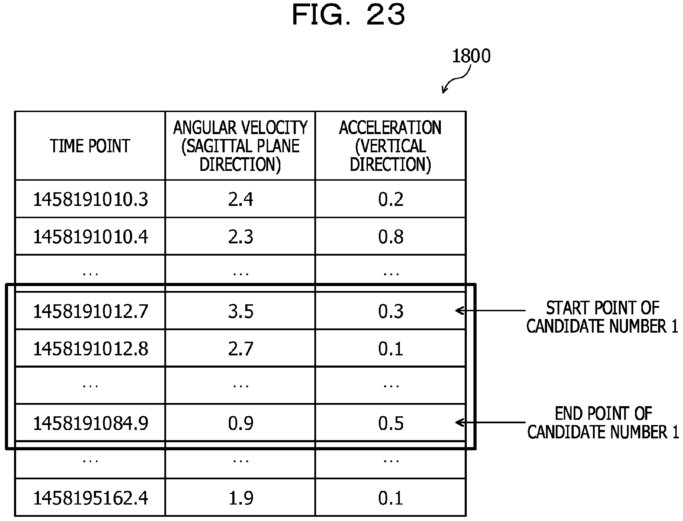

In FIG. 22, the information processing apparatus 100 allocates a candidate period number for each candidate period. Then, the information processing apparatus 100 stores a record, in which the candidate period number of the candidate period, a start point of the candidate period, and an end point of the candidate period are associated with each other, by using a candidate period management table 2200. Next, description of FIG. 23 will be made.

In FIG. 23, the information processing apparatus 100 selects a candidate period having a candidate number 1. The information processing apparatus 100 extracts gyro data for the selected candidate period from the right shin data 1800. Similarly, the information processing apparatus 100 extracts gyro data for the selected candidate period from the left shin data 1900.

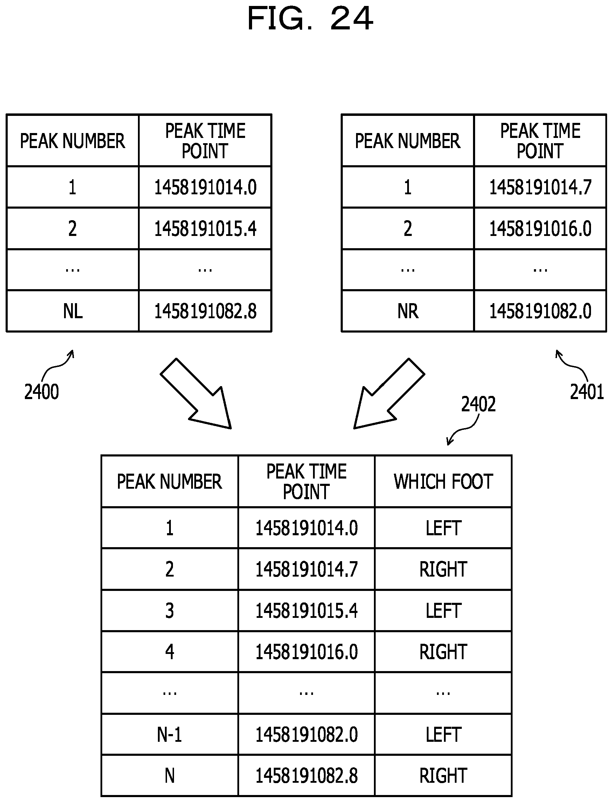

The information processing apparatus 100 specifies a peak point regarding the left shin and a peak point regarding the right shin based on the extracted gyro data. For example, after applying a low-pass filter to the extracted gyro data, the information processing apparatus 100 specifies a peak point, at which the angular velocity exceeds a threshold and becomes a local maximum value, having an interval with the other peak point equal to or more than a certain value. Next, description of FIG. 24 will be made.

In FIG. 24, the information processing apparatus 100 allocates a peak number for each specified peak point regarding the right shin. Then, the information processing apparatus 100 stores a record in which a peak number of a peak point regarding the right shin is associated with a time point indicated by the peak point regarding the right shin by using a right shin peak point management table 2400.

Similarly, the information processing apparatus 100 allocates a peak number for each specified peak point regarding the left shin. Then, the information processing apparatus 100 stores a record in which the peak number of the peak point regarding the left shin is associated with a time point indicated by the peak point regarding the left shin by using a left shin peak point management table 2401.

Then, the information processing apparatus 100 refers to the right shin peak point management table 2400 and the left shin peak point management table 2401 and sorts the peak points regarding the right shin and the peak points regarding the left shin together in the order of occurrence.

The information processing apparatus 100 allocates a peak number to the peak point regarding the right shin and the peak point regarding the left shin in the order of occurrence. Then, the information processing apparatus 100 stores a record in which the peak number of the peak point regarding each shin is associated with a time point indicated by the peak point regarding each shin by using an each shin peak point management table 2402.

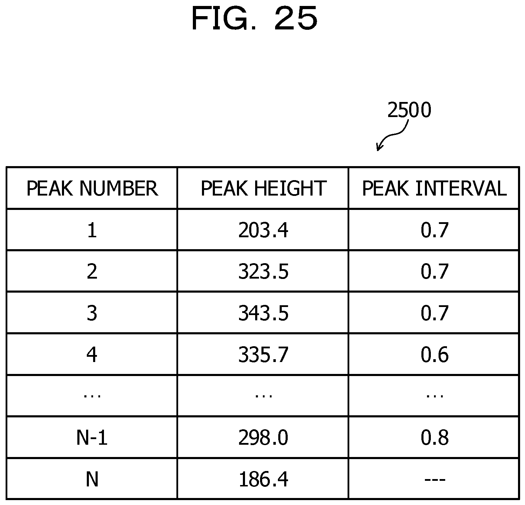

The information processing apparatus 100 refers to the each shin peak point management table 2402 and determines whether or not the peak points regarding the respective shins alternately appear. In a case where the peak points regarding the respective shins alternately appear, the information processing apparatus 100 specifies the selected candidate period as the walking period. Next, description of FIG. 25 will be made.

In FIG. 25, the information processing apparatus 100 refers to the each shin peak point management table 2402 and calculates a feature quantity regarding a motion of each shin at the peak point regarding each shin. The feature quantity is, for example, a peak height or a peak interval. The peak height is a magnitude of the angular velocity at the peak point. The peak interval is a time to the next peak point.

The information processing apparatus 100 stores a record in which the peak number of the peak point regarding each shin is associated with the peak height and the peak interval at the peak point by using a feature quantity management table 2500. The information processing apparatus 100 refers to the feature quantity management table 2500 and calculates variations in the peak height and the peak interval. The variation is, for example, dispersion.

When the variation in the peak height is equal to or less than a threshold and the variation in the peak interval is equal to or less than a threshold, the information processing apparatus 100 determines that the subject tends to stably walk in the candidate period. In the following description, there is a case where to tend to stably walk is referred to as "stable walking ability".

Furthermore, the information processing apparatus 100 may determine whether or not the subject tends to stably walk in the candidate period by using a spectrum distribution in addition to the variations in the peak height and the peak interval. Specifically, the information processing apparatus 100 calculates an evaluation value H that increases as the number of peaks of the frequency spectrum is smaller and the intensity distribution is uneven, by using the following formula (14). Since the following formula (14) is similar to the above formula (7), description thereof will be omitted.

[Expression 14]

$$H = - \sum_{i=1}^{M} p_i \log p_i \quad \dots (14)$$

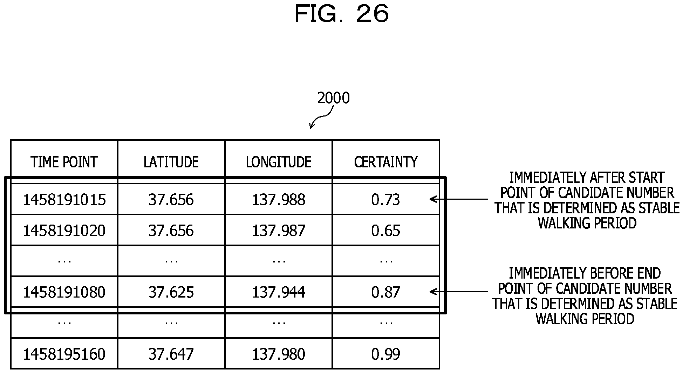

Then, when H is equal to or more than a threshold, the information processing apparatus 100 determines that the subject tends to stably walk in the candidate period. Next, the information processing apparatus 100 determines whether or not the subject tends to stably move in the candidate period. In the following description, there is a case where to tend to stably move is referred to as "stable moving ability". Here, description of FIG. 26 will be made.

In FIG. 26, the information processing apparatus 100 extracts position information of a part corresponding to the candidate period from the position information 2000. The information processing apparatus 100 specifies GPS coordinates of the subject for each observation point in the candidate period based on the extracted position information and determines whether or not the candidate period is a period in which the subject moves straight and the position of the subject tends to change at equal intervals. When the subject moves straight and the position of the subject tends to change at equal intervals, the information processing apparatus 100 determines that the subject tends to stably move.

The information processing apparatus 100 specifically calculates the variation $E_1$ in the intervals of the observation points by using the following formula (15). Then, when the variation $E_1$ is equal to or less than a threshold, the information processing apparatus 100 determines that the position of the subject tends to change at the equal intervals.

[Expression 15]

$$E_1 = \operatorname*{var}_{1 < n < N} (|d_n|) \quad \dots (15)$$

Furthermore, the information processing apparatus 100 specifically calculates the angle $E_2$ formed by a travel vector $d_n$ from an observation point to a next observation point by using the following formula (16). Then, when the angle $E_2$ is equal to or less than a threshold, the information processing apparatus 100 determines that the subject tends to move straight.

[Expression 16]

$$E_2 = \frac{1}{N}\left|\sum_{n=1}^{N} \theta_n\right| = \frac{1}{N}\left|\sum_{n=1}^{N} arccos\left(\frac{d_{n+1} \cdot d_n}{|d_{n+1}| \times |d_n|}\right)\right| \quad ...(16)$$

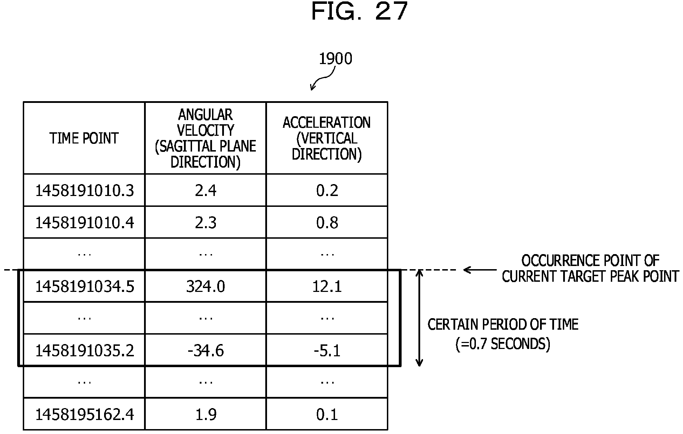

Then, in a case of determining that the subject tends to stably walk and tends to stably move in the candidate period, the information processing apparatus 100 sets the candidate period as the stable walking period. Next, description of FIGs. 27 to 29 will be made, and a specific example in which the information processing apparatus 100 specifies the both-leg grounding period will be described.

FIGs. 27 to 29 are explanatory diagrams illustrating a specific example in which the information processing apparatus 100 specifies the both-leg grounding period. In FIG. 27, the information processing apparatus 100 refers to the each shin peak point management table 2402 and selects the peak points one by one. The information processing apparatus 100 specifies a heel-strike point so as to specify a support period corresponding to the selected peak point.

As illustrated in FIG. 27, specifically, in a case of selecting the peak point regarding the left shin, the information processing apparatus 100 specifies a time point, in the left shin data 1900, when the angular velocity on the sagittal plane becomes the local minimum value within a certain period of time after the peak point as the heel-strike point.

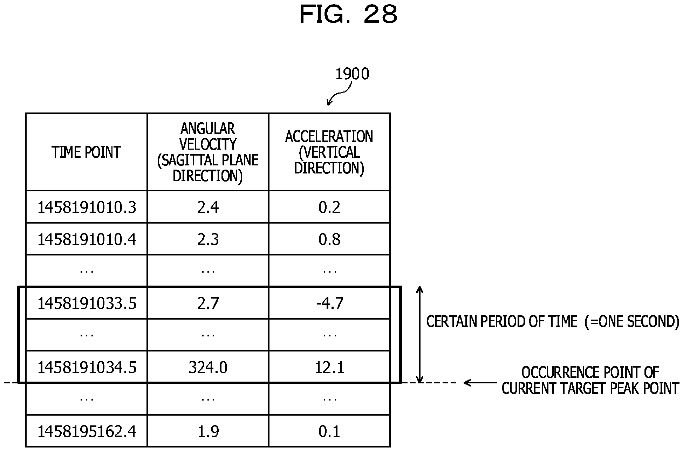

Furthermore, since the measuring instrument 201 receives a reaction force from the direction of gravity at the heel-strike point, the information processing apparatus 100 may specify a time point when the acceleration in the vertical direction changes from minus to plus most significantly within the certain period of time after the peak point as the heel-strike point. Next, description of FIG. 28 will be made.

In FIG. 28, the information processing apparatus 100 refers to the each shin peak point management table 2402 and selects the peak points one by one. The information processing apparatus 100 specifies a toe-off point so as to specify a support period corresponding to the selected peak point.

As illustrated in FIG. 28, specifically, in a case of selecting the peak point regarding the left shin, the information processing apparatus 100 specifies a time point, in the left shin data 1900, when the angular velocity on the sagittal plane becomes the local minimum value within a certain period of time before the peak point as the toe-off point.

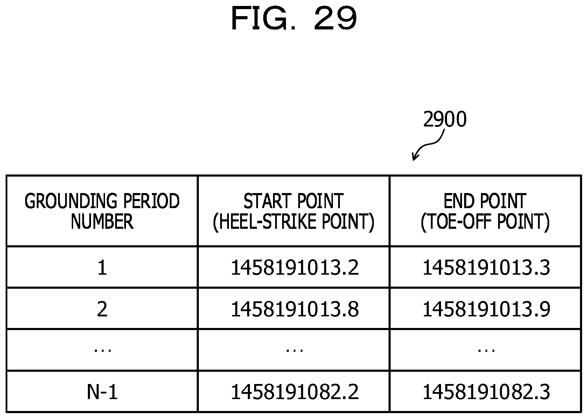

Furthermore, since the information processing apparatus 100 receives a reaction force at the toe-off point from the direction of gravity to a direction opposite to that at the heel-strike point, the information processing apparatus 100 may specify a time point when the acceleration in the vertical direction changes from plus to minus most significantly within the certain period of time before the peak point as the toe-off point. Next, description of FIG. 29 will be made.

In FIG. 29, the information processing apparatus 100 specifies a period from the heel-strike point to the toe-off point as the support period. The information processing apparatus 100 specifies a period in which different support periods overlap as the both-leg grounding period.

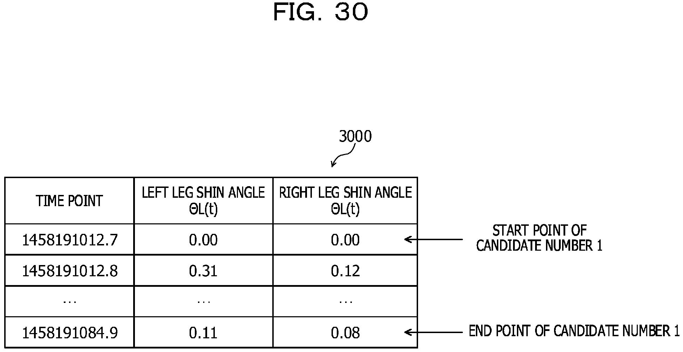

The information processing apparatus 100 allocates a grounding period number to the specified both-leg grounding period. For each both-leg grounding period, the information processing apparatus 100 stores a record, in which the grounding period number allocated to the both-leg grounding period, a start point of the both-leg grounding period, and an end point of the both-leg grounding period are associated with each other, by using a both-leg grounding period management table 2900. Next, description of FIGs. 30 to 32 will be made, and a specific example in which the information processing apparatus 100 calculates a shin angle will be described.

FIGs. 30 to 33 are explanatory diagrams illustrating a specific example in which the information processing apparatus 100 calculates the shin angle. In FIG. 30, the information processing apparatus 100 calculates each of the shin angles $\theta_L$ and $\theta_R$ by using the following formula (17) based on gyro data of each shin for each specified both-leg grounding period.

[Expression 17]

$$\theta(t) = \theta_0 + \sum_{i=0}^{t} gyro(i) \quad ...(17)$$

Here, the reference $\theta(t)$ is a shin angle. The reference $\theta_0$ is an initial value of the shin angle set as an initial state. The reference gyro(i) is an angular velocity of the shin at a time point i.

Accordingly, the information processing apparatus 100, for example, sets the initial value $\theta_0$ of the shin angle to zero at the head of the stable walking period. Then, the information processing apparatus 100 calculates each of the shin angles $\theta_L$ and $\theta_R$ at each time point by adding an integral value of the angular velocity of each shin before each time point to $\theta_0$ based on the gyro data of each shin.

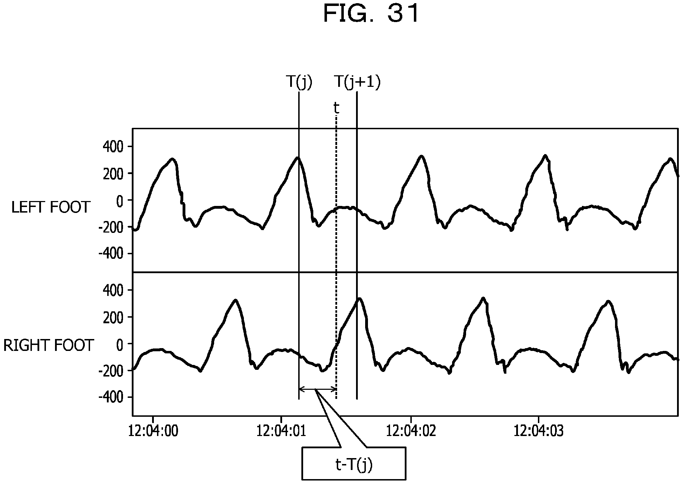

Then, the information processing apparatus 100 associates the time points when the respective shin angles $\theta_L$ and $\theta_R$ are calculated with the respective shin angles $\theta_L$ and $\theta_R$ and stores the associated value by using a shin angle management table 3000. Next, description of FIG. 31 will be made, and another specific example in which the information processing apparatus 100 calculates the shin angle will be described.

In FIG. 31, the information processing apparatus 100 calculates each of the shin angles $\theta_L$ and $\theta_R$ in consideration of an error included in the gyro data and improves accuracy of calculating each of the shin angles $\theta_L$ and $\theta_R$. Here, the error in the gyro data tends to increase as time elapses from the start of measurement.

Therefore, in a case where the subject stably walks, it is assumed that the shin angles tend to coincide with each other at a predetermined kind of time point for each step. Therefore, the information processing apparatus 100 calculates each of the shin angles $\theta_L$ and $\theta_R$ after correcting the initial value indicating the shin angle in the initial state for each step. The predetermined kind of time peak point is, for example, the heel-strike point, the toe-off point, the peak point, and the like. For example, the information processing apparatus 100 calculates each of the shin angles $\theta_L$ and $\theta_R$ for each step by using the following formula (18).

[Expression 18]

$$\theta(t) = \theta_0\big(T(j)\big) + \sum_{i=T(j)}^{t} [gyro(i) + \varepsilon] \quad \text{...}(18)$$

Here, the reference $\theta(t)$ is a shin angle at the time point t. The reference $\theta_0(T(j))$ is an initial value of a shin angle set at the predetermined kind of time point in a j-th step. Regarding $\theta_0(T(j))$, a shin angle at a predetermined kind of time point in a first step may be set as an initial value. The reference gyro(i) is an angular velocity of the shin at a time point i. The reference $\varepsilon$ is an error with respect to the gyro data.

Here, at a head and an end of the stable walking period, the subject tends to be in the stationary state, and the shin angles of the subject tend to coincide with each other. Therefore, the information processing apparatus 100 specifies the error $\varepsilon$ by using the following formulas (19) and (20).

[Expression 19]

$$\theta_1 = \theta_0 \quad \text{...}(19)$$

[Expression 20]

$$\theta_2 = \theta_0 + \sum_{i=T_s}^{T_e} [gyro(i) + \varepsilon] \quad \text{...}(20)$$

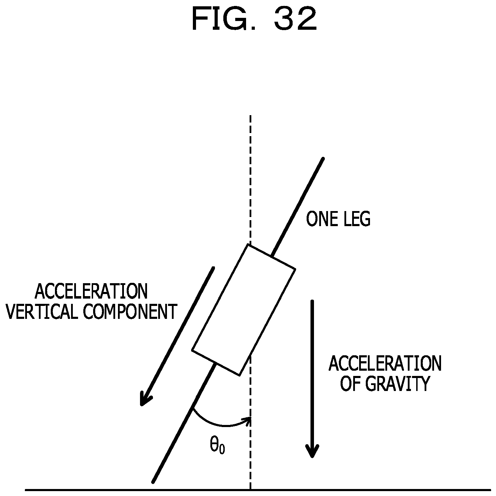

Here, the reference $\theta_1$ is a shin angle at a head $T_s$ of the stable walking period. The reference $\theta_2$ is a shin angle at an end $T_e$ of the stable walking period. Furthermore, if a time point when the subject has the same posture can be specified from the stable walking period, the information processing apparatus 100 can use the specified time point instead of the head $T_s$ of the stable walking period and the end $T_e$ of the stable walking period. Next, description of FIG. 32 will be made.

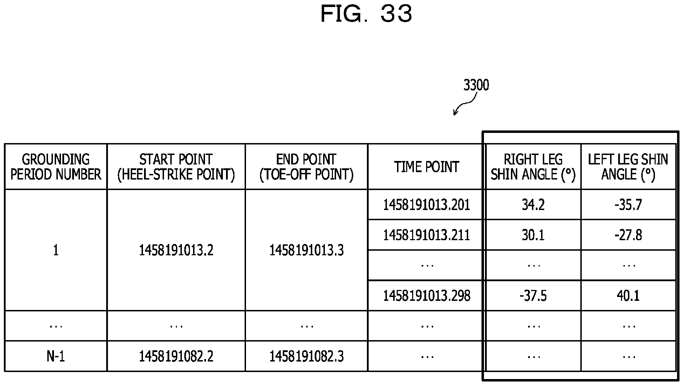

In FIG. 32, the information processing apparatus 100 may set the initial value $\theta_0$ of the shin angle based on the acceleration data, not zero. Here, a value of an acceleration AccZ in a direction along the lower leg of the subject, that is, the vertical direction, at the head of the stable walking period has a property to be a value obtained by multiplying the acceleration of gravity with $\cos\theta_0$. Therefore, the information processing apparatus 100 can set the initial value $\theta_0$ of the shin angle. Next, description of FIG. 33 will be made.

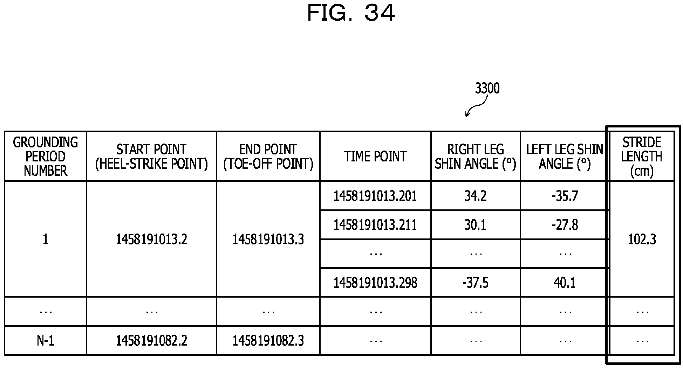

In FIG. 33, the information processing apparatus 100 calculates each of the shin angles $\theta_L$ and $\theta_R$ at each time point in the both-leg grounding period. The information processing apparatus 100 stores a record in which the both-leg grounding period, each time point in the both-leg grounding period, and the shin angles $\theta_L$ and $\theta_R$ calculated for each time point are associated with each other by using an information table for calculation 3300. Next, description of FIG. 34 will be made, and a specific example in which the information processing apparatus 100 calculates the stride length will be described.

FIG. 34 is an explanatory diagram illustrating the specific example in which the information processing apparatus 100 calculates the stride length. In FIG. 34, the information processing apparatus 100 calculates a movement distance D of the subject in the stable walking period based on the position information. Furthermore, the information processing apparatus 100 calculates the number of peak points in the stable walking period as the number of steps N in the stable walking period.

Here, in the stable walking period, the subject tends to stably move and stably walk, and the stride length of the subject tends to be constant. Therefore, the information processing apparatus 100 calculates the estimated stride length SL for each step by using the following formula (21).

[Expression 21]

$$SL = \frac{D}{N} \quad \cdots (21)$$

The information processing apparatus 100 stores the calculated estimated stride length SL by using the information table for calculation 3300. Furthermore, at the first step and the final step of the stable walking period, the subject tends to move only half a step. Therefore, the information processing apparatus 100 may calculate the stride length SL by dividing the movement distance D by the number of steps N-1. In this case, the information processing apparatus 100 sets SL/2 to the stride length of the first step or the final step.

Furthermore, there is a possibility that the stride length is not constant. Therefore, the information processing apparatus 100 may calculate a feature quantity correlated with the stride length for each step and weight the stride length by using the feature quantity. For example, the information processing apparatus 100 calculates a movement distance, as assuming that a feature quantity in the i-th step be f(i), by using the following formula (22).

[Expression 22]

$$D = \sum_{i=1}^{N} \eta f(i) \quad \cdots (22)$$

The above formula (22) can be modified to the following formula (23).

[Expression 23]

$$\eta = \frac{D}{\sum_{i=1}^{N} f(i)} \quad \cdots (23)$$

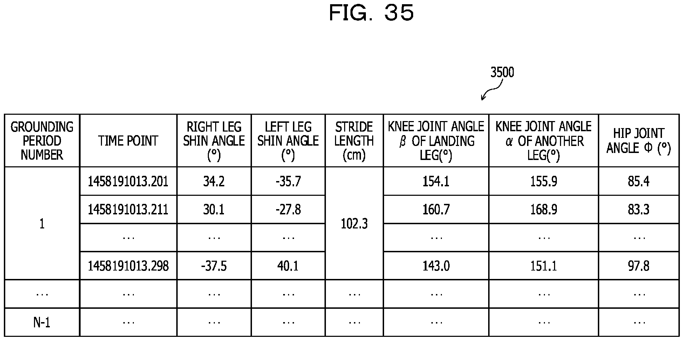

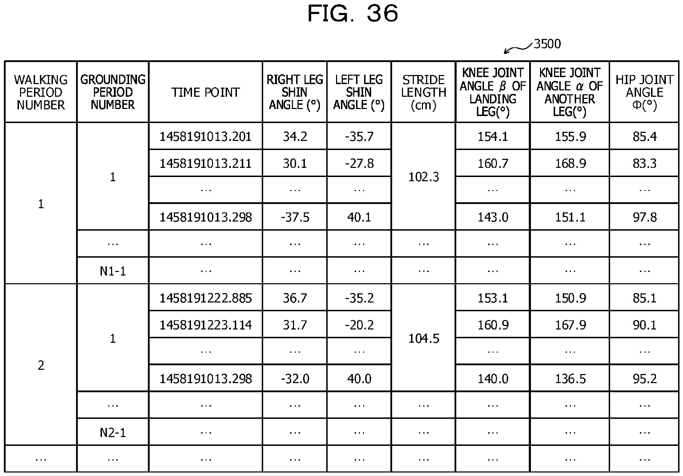

The information processing apparatus 100 calculates the stride length of the i-th step as $\eta \times f(i)$ by using the above formula (23). The feature quantity is, for example, a height of the peak point, an interval between the peak points, and the like. Next, description of FIGs. 35 and 36 will be made, and a specific example in which the information processing apparatus 100 calculates each knee joint angle and the hip joint angle will be described.

FIGs. 35 and 36 are explanatory diagrams illustrating the specific example in which the information processing apparatus 100 calculates each knee joint angle and the hip joint angle. In FIG. 35, the information processing apparatus 100 calculates the knee joint angles $\alpha$ and $\beta$ and the hip joint angles $\varphi_L$ and $\varphi_R$ by using the following formulas (24) to (27) based on the calculated estimated stride length SL and the calculated shin angles $\theta_L$ and $\theta_R$. Since the following formulas (24) to (27) are similar to the above formulas (1) to (4), description thereof will be omitted.

[Expression 24]

$$SL = L\cos\theta_L + L\sin\phi_L + L\sin\phi_R + L\cos\theta_R \quad \cdots (24)$$

[Expression 25]

$$h = L \sin \theta_L + L \cos \phi_L = L \sin \theta_R + L \cos \phi_R \quad ...(25)$$

[Expression 26]

$$\alpha = \frac{\pi}{2} + \theta_L + \phi_L \quad ...(26)$$

[Expression 27]

$$\beta = \frac{\pi}{2} + \theta_R - \phi_R \quad ...(27)$$

With this calculation, he information processing apparatus 100 can calculate the knee joint angles $\alpha$ and $\beta$ and the hip joint angles $\varphi_L$ and $\varphi_R$. Moreover, the information processing apparatus 100 calculates the hip joint angle $\varphi$ based on the hip joint angles $\varphi_L$ and $\varphi_R$ by using the following formula (28).
[Expression 28]

$$\phi = \phi_R + \phi_L \quad ...(28)$$

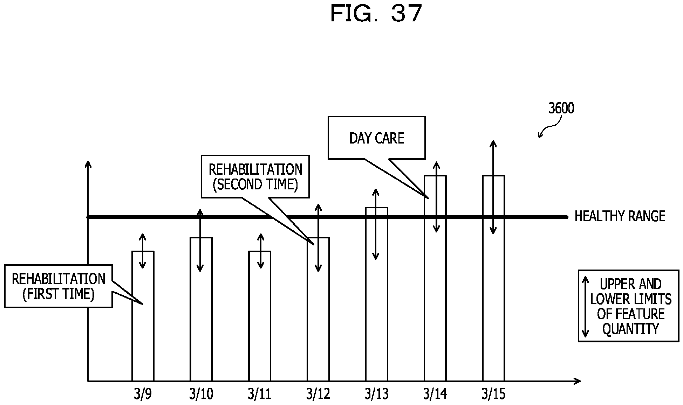

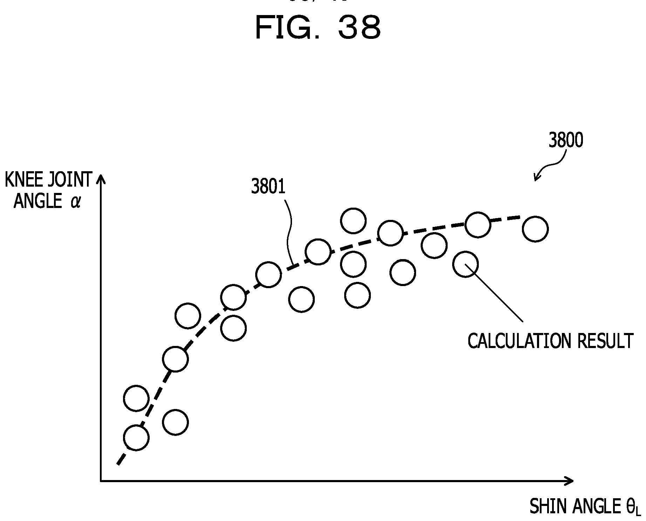

With this calculation, the information processing apparatus 100 can calculate the hip joint angle $\varphi$. The information processing apparatus 100 stores the calculated knee joint angles $\alpha$ and $\beta$ and hip joint angle $\varphi$ by using a calculation result table 3500. Then, since it is not necessary for the information processing apparatus 100 to directly calculate the angle other than the shin angle from the gyro data, an increase in the number of measuring instruments 201 attached to the patient can be reduced.
Furthermore, the information processing apparatus 100 can specify the stable walking period and calculate the knee joint angles $\alpha$ and $\beta$ and the hip joint angle $\varphi$ in the stable walking period. Therefore, the information processing apparatus 100 can prevent an adverse effect on the calculation result due to unstable walking and improve the accuracy of calculating the knee joint angles $\alpha$ and $\beta$ and the hip joint angle $\varphi$. Next, description of FIG. 36 will be made.
Similarly, in FIG. 36, the information processing apparatus 100 stores the knee joint angles $\alpha$ and $\beta$ and the hip joint angle $\varphi$ calculated for each specified stable walking period by using the calculation result table 3500. Next, description of FIG. 37 will be made.
FIG. 37 is an explanatory diagram illustrating an exemplary output. In FIG. 37, the information processing apparatus 100 specifies tendency of the knee joint angles and the hip joint angle of the patient based on the calculated knee joint angles and hip joint angle and outputs the specified tendency. For example, the information processing apparatus 100 receives a designation of a target data range and reads a knee joint angle and a hip joint angle in the data range. Then, to make it possible to grasp transition of a daily walking form of the patient, the information processing apparatus 100 calculates statistical values of the knee joint angle and the hip joint angle for each date and displays the statistical values as in a graph 3600.
Here, the information processing apparatus 100 may display a range to be a reference value in a case where the subject is healthy, on the graph 3600. Furthermore, the information processing apparatus 100 may display a timing when a reference event related to the patient, such as medical care of the patient in the future, is held on the graph 3600. Furthermore, the information processing apparatus 100 may display a range from an upper limit to a lower limit of each of the knee joint angle and the hip joint angle of the patient for each date on the graph 3600. Next, description of FIG. 38 will be made, and a specific example in which the information processing apparatus 100 uses a calculation result will be described.
FIG. 38 is an explanatory diagram illustrating the specific example in which the information processing apparatus 100 uses the calculation result. According to the processing illustrated in FIGs. 14 to 38, the information processing apparatus 100 can specify a plurality of combinations of the shin angles $\theta_L$ and $\theta_R$ and the knee joint angles $\alpha$ and $\beta$ and the hip joint angles $\varphi_L$ and $\varphi_R$ respectively corresponding to the shin angles $\theta_L$ and $\theta_R$. Therefore, the information processing apparatus 100 can define a relationship between the angle $\theta_L$ of one shin and the knee joint angle $\alpha$ and the hip joint angle $\varphi_L$ corresponding to the angle $\theta_L$ of the shin by a function.
A graph 3800 in FIG. 38 indicates the relationship between the angle $\theta_L$ of the shin and the knee joint angle $\alpha$ corresponding to the angle $\theta_L$ of the shin. The horizontal axis indicates the angle $\theta_L$, and the vertical axis indicates the

knee joint angle $\alpha$. For example, the information processing apparatus 100 generates a function indicating an approximate curve 3801 from the calculation result. Accordingly, even if the processing illustrated in FIGs. 14 to 38 is not performed, the information processing apparatus 100 can calculate the knee joint angles $\alpha$ and $\beta$ and the hip joint angles $\varphi_L$ and $\varphi_R$ respectively corresponding to the angles $\theta_L$ and $\theta_R$ of the respective shins based on the angles $\theta_L$ and $\theta_R$ of the respective shins.

Therefore, the information processing apparatus 100 can reduce time required when each of the knee joint angles $\alpha$ and $\beta$ and hip joint angles $\varphi_L$ and $\varphi_R$ is calculated. Furthermore, even if the position information is not acquired, the information processing apparatus 100 can calculate each of the knee joint angles $\alpha$ and $\beta$ and the hip joint angles $\varphi_L$ and $\varphi_R$.

Thereafter, according to the processing illustrated in FIGs. 14 to 38, the information processing apparatus 100 can specify the plurality of combinations of the shin angles $\theta_L$ and $\theta_R$ and the knee joint angles $\alpha$ and $\beta$ and the hip joint angles $\varphi_L$ and $\varphi_R$ respectively corresponding to the shin angles $\theta_L$ and $\theta_R$ at constant intervals again. Then, the information processing apparatus 100 can update the approximate curve 3801 and prevent deterioration in the accuracy of calculating each of the knee joint angles $\alpha$ and $\beta$ and the hip joint angles $\varphi_L$ and $\varphi_R$.

(Exemplary Angle Calculation Processing Procedure)

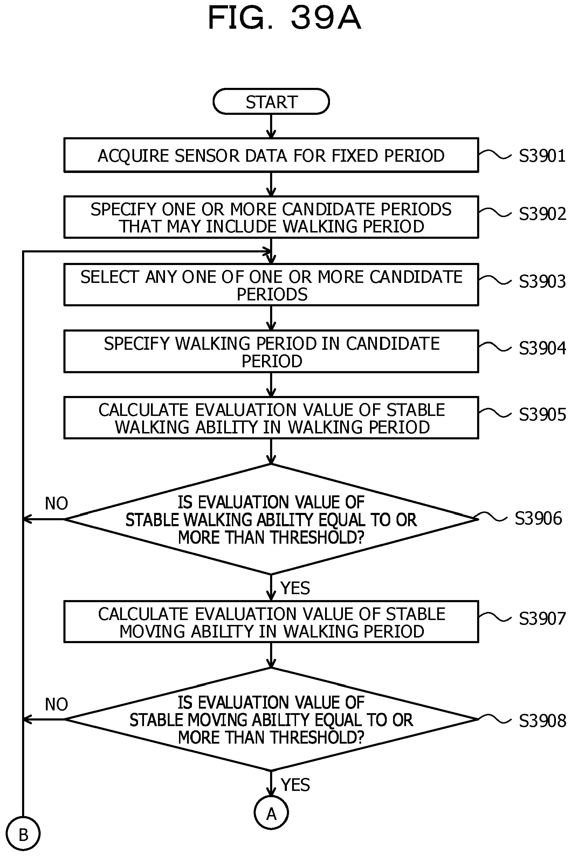

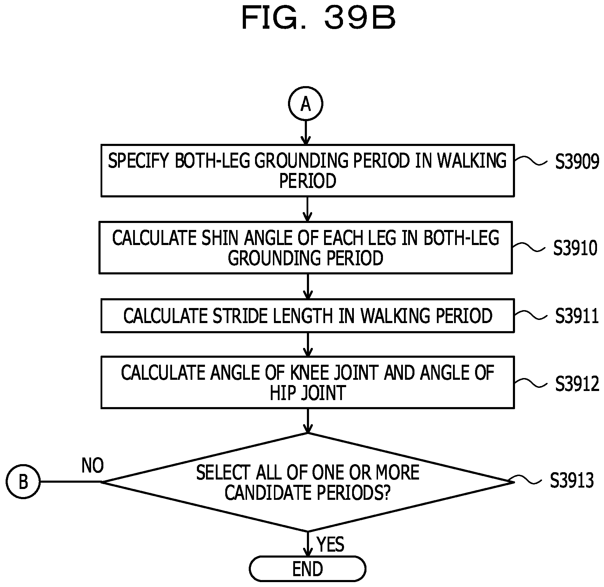

Next, an exemplary angle calculation processing procedure executed by the information processing apparatus 100 will be described with reference to FIG. 39.

FIG. 39 is a flowchart illustrating an exemplary angle calculation processing procedure. In FIG. 39, first, the information processing apparatus 100 acquires sensor data for a fixed period from the measuring instrument 201 (step S3901).

Next, the information processing apparatus 100 specifies one or more candidate periods that may include the walking period based on the acquired sensor data (step S3902). Then, the information processing apparatus 100 selects any one of the specified one or more candidate periods (step S3903).

Next, the information processing apparatus 100 specifies the walking period in the selected candidate period (step S3904). Then, the information processing apparatus 100 calculates an evaluation value of a stable walking ability in the specified walking period (step S3905) .

Next, the information processing apparatus 100 determines whether or not the evaluation value of the stable walking ability is equal to or more than a threshold (step S3906). Here, in a case where the evaluation value is less than the threshold (No in step S3906), the information processing apparatus 100 returns to the processing in step S3903.

On the other hand, in a case where the evaluation value is equal to or more than the threshold (Yes in step S3906), the information processing apparatus 100 calculates the evaluation value of the stable moving ability in the specified walking period (step S3907).

Next, the information processing apparatus 100 determines whether or not an evaluation value of the stable moving ability is equal to or more than a threshold (step S3908). Here, in a case where the evaluation value is less than the threshold (No in step S3908), the information processing apparatus 100 returns to the processing in step S3903.

On the other hand, in a case where the evaluation value is equal to or more than the threshold (Yes in step S3908), the information processing apparatus 100 specifies the both-leg grounding period in the walking period (step S3909). Next, the information processing apparatus 100 calculates a shin angle of each leg in the specified both-leg grounding period (step S3910). Then, the information processing apparatus 100 calculates a stride length in the walking period (step S3911).

Next, the information processing apparatus 100 calculates angles of the knee joint and the hip joint (step S3912). Then, the information processing apparatus 100 determines whether or not all the one or more specified candidate periods have been selected (step S3913). Here, in a case where there is a candidate period that has not been selected (No in step S3913), the information processing apparatus 100 returns to the processing of step S3903.

On the other hand, in a case where all the candidate periods have been selected (Yes in step S3913), the information processing apparatus 100 ends the angle calculation processing.

As described above, according to the information processing apparatus 100, the information regarding the stride length of the subject and the information regarding the angle of each lower leg of the subject with respect to the ground in a case where both feet of the subject are grounded can be acquired. According to the information processing apparatus 100, the angles of the respective knee joints of the subject and the angle of the hip joint of the subject that satisfy the geometric constraints, derived from the structure of the lower limbs of the subject in a case where both feet of the subject are grounded can be calculated based on the information regarding the stride length and the information regarding the angles that are acquired. With this calculation, the information processing apparatus 100 can improve the accuracy of calculating the angle of each knee joint of the subject and the angle of the hip joint of the subject.

According to the information processing apparatus 100, the geometric constraint can be defined by the simultaneous equation including the expression indicating the first constraint and the expression indicating the second constraint, and the angle of each of the knee joints of the subject and the angle of the hip joint of the subject can be calculated by solving the simultaneous equation. With this calculation, the information processing apparatus 100 can define the geometric constraint by a relatively easy simultaneous equation and solve the simultaneous equation at relatively high speed.

According to the information processing apparatus 100, the movement distance of the subject and the number of steps of the subject in the predetermined walking period can be acquired. According to the information processing apparatus 100, the stride length of the subject can be calculated by dividing the acquired movement distance by the number of steps. Accordingly, the information processing apparatus 100 can acquire the stride length of the subject even in a case where the stride length of the subject cannot be directly measured.

According to the information processing apparatus 100, the angular velocity of the sagittal plane of each lower leg of the subject in the predetermined walking period can be acquired. According to the information processing apparatus 100, by adding an integral value of the angular velocity acquired before the grounding time when both feet of the subject are grounded to the angle of each lower leg of the subject with respect to the ground at the time when the subject is stationary, the angle of each lower leg of the subject with respect to the ground at the grounding time can be calculated. Accordingly, the information processing apparatus 100 can acquire the angle of each lower leg of the subject with respect to the ground even in a case where the angle of each lower leg of the subject with respect to the ground cannot be directly measured.

According to the information processing apparatus 100, the acceleration in the direction along the lower leg of the subject at the time when the subject is stationary that is the head of the predetermined walking period can be acquired. According to the information processing apparatus 100, the angle of each lower leg of the subject with respect to the ground at the time when the subject is stationary that is the head of the predetermined walking period can be calculated based on the acquired acceleration in the direction along the lower leg of the subject and the acceleration of gravity. With this calculation, the information processing apparatus 100 can improve the accuracy of calculating the angle of each lower leg of the subject with respect to the ground at the grounding time.

According to the information processing apparatus 100, the plurality of motion time points, in the predetermined walking period, when the feature corresponding to one step of the subject appears in the acquired angular velocity or acceleration of the subject can be specified. According to the information processing apparatus 100, the angle of each lower leg of the subject with respect to the ground at the first motion time point can be calculated. According to the information processing apparatus 100, the integral value of the angular velocity acquired from the motion time point that is closest to and immediately before the grounding time of the plurality of motion time points to the grounding time is added to the predetermined angle of each lower leg of the subject with respect to the ground at the first motion time point. With this calculation, the information processing apparatus 100 can improve the accuracy of calculating the angle of each lower leg of the subject with respect to the ground at the grounding time.

According to the information processing apparatus 100, an error relative to the angular velocity of the sagittal plane of each lower leg of the subject in the predetermined walking period can be specified so that the integral value of the angular velocity of the sagittal plane of each lower leg of the subject before a final time point of the predetermined walking period becomes zero. According to the information processing apparatus 100, the angle of each lower leg of the subject with respect to the ground at the grounding time can be calculated based on the specified error. With this calculation, the information processing apparatus 100 can improve the accuracy of calculating the angle of each lower leg of the subject with respect to the ground at the grounding time.

According to the information processing apparatus 100, it is possible to acquire the information indicating the moving speed and the moving direction of the subject from the measuring instrument and set the stable period in which the moving speed and the moving direction are constant as the predetermined walking period. With this operation, the information processing apparatus 100 can set the stable period in which the subject stably moves as the predetermined walking period and can improve the accuracy of calculating the angle of each knee joint of the subject and the angle of the hip joint of the subject.

According to the information processing apparatus 100, the plurality of motion time points, in the predetermined candidate period, when the feature corresponding to one step of the subject appears in the angular velocity or acceleration of the subject can be specified. According to the information processing apparatus 100, the stable period, in which the intervals of the motion time points are constant, can be set as the predetermined walking period in the predetermined candidate period based on the plurality of specified motion time points. With this operation, the information processing apparatus 100 can set the stable period in which the subject stably walks as the predetermined walking period and can improve the accuracy of calculating the angle of each knee joint of the subject and the angle of the hip joint of the subject.

According to the information processing apparatus 100, the plurality of motion time points, in the predetermined candidate period, when the feature corresponding to one step of the subject appears in the angular velocity or acceleration of the subject can be specified. According to the information processing apparatus 100, the stable period, in which the feature quantity of the motion of the subject at the motion time point is constant can be set as the predetermined walking period based on the plurality of specified motion time points in the predetermined candidate period. With this operation, the information processing apparatus 100 can set the stable period in which the subject stably moves as the predetermined walking period and can improve the accuracy of calculating the angle of each knee joint of the subject and the angle of the hip joint of the subject.

Note that the information processing method described in the present embodiment can be implemented by executing a prepared program on a computer such as a personal computer or a workstation. The information processing program described in the present embodiment is recorded on a computer-readable recording medium such as a hard disk, flexible disk, compact disk read only memory (CD-ROM), magneto-optical disk (MO), or digital versatile disc (DVD), and is read from the recording medium to be executed by the computer. Furthermore, the information processing program described in the present embodiment may be distributed via a network such as the Internet.

REFERENCE SIGNS LIST

| | |
|---|---|
| 100 | information processing apparatus |
| 110 | polygon |
| 200 | information processing system |
| 201 | measuring instrument |
| 210 | network |
| 300, 400 | bus |
| 301, 401 | CPU |
| 302, 402 | memory |
| 303, 403 | network I/F |
| 304 | recording medium I/F |
| 305 | recording medium |
| 404 | sensor unit |
| 405 | timer unit |
| 500 | storage unit |
| 501 | acquisition unit |
| 502 | specification unit |
| 503 | calculation unit |
| 504 | output unit |
| 700 | perpendicular |
| 800, 1100, 1101, 1300, 3600, 3800 | graph |
| 801 to 804, 811 to 815 | peak point |
| 820, 1000, 1001 | period |
| 900, 901 | route |
| 1301, 3000 | shin angle management table |
| 1800 | right shin data |
| 1900 | left shin data |
| 2000 | position information |
| 2200 | candidate period management table |
| 2400 | right shin peak point management table |
| 2401 | left shin peak point management table |
| 2402 | each shin peak point management table |
| 2500 | feature quantity management table |
| 2900 | both-leg grounding period management table |
| 3300 | information table for calculation |
| 3500 | calculation result table |
| 3801 | approximate curve |

**Claims**

**1.** An information processing apparatus comprising:

a controller configured to
acquire information regarding a stride length of a subject and information regarding an angle of each lower leg of the subject with respect to a ground in a case where both feet of the subject are grounded, and
calculate at least any one of an angle of each knee joint of the subject and an angle of a hip joint of the subject that satisfy a geometric constraint derived from a structure of lower limbs of the subject in a case where both feet of the subject are grounded based on the acquired information regarding the stride length and the angle.

2. The information processing apparatus according to claim 1, wherein

the geometric constraint includes
a first constraint indicating a relationship between the stride length of the subject, a length of a thigh of the subject, a length of a lower leg of the subject, the angle of each knee joint of the subject, and the angle of the hip joint of the subject, and
a second constraint indicating a relationship between a waist height of the subject calculated based on the length of the thigh of the subject, the length of the lower leg of the subject, an angle of a right knee joint of the subject, and the angle of the hip joint of the subject, and a waist height of the subject calculated based on the length of the thigh of the subject, the length of the lower leg of the subject, an angle of a left knee joint of the subject, and the angle of the hip joint of the subject.

3. The information processing apparatus according to claim 1 or 2, wherein

the controller
acquires a movement distance of the subject and the number of steps of the subject in a predetermined walking period, and
calculates the stride length of the subject by dividing the acquired movement distance by the number of steps.

4. The information processing apparatus according to any one of claims 1 to 3, wherein

the controller
acquires an angular velocity of a sagittal plane of each lower leg of the subject in the predetermined walking period from a measuring instrument that measures the angular velocity of the sagittal plane of each lower leg of the subject, and
calculates the angle of each lower leg of the subject with respect to the ground at a grounding time by adding an integral value of the angular velocity acquired before the grounding time when both feet of the subject are grounded to the angle of each lower leg of the subject with respect to the ground at a time when the subject is stationary.

5. The information processing apparatus according to claim 4, wherein

the controller
acquires an acceleration in a direction along the lower leg of the subject at the time when the subject is stationary that is a head of the predetermined walking period from a measuring instrument that measures the acceleration in the direction along the lower leg of the subject, and
calculates the angle of each lower leg of the subject with respect to the ground at the time when the subject is stationary that is the head of the predetermined walking period based on the acquired acceleration in the direction along the lower leg of the subject and an acceleration of gravity.

6. The information processing apparatus according to claim 4 or 5, wherein

the controller
acquires an angular velocity or an acceleration of the subject in the predetermined walking period from a measuring instrument that measures the angular velocity or the acceleration of the subject,
specifies a plurality of motion time points, in the predetermined walking period, when a feature corresponding to one step of the subject appears based on the acquired angular velocity or acceleration of the subject,
calculates an angle of each lower leg of the subject with respect to the ground at a first motion time point by adding an integral value of the angular velocity acquired before the first motion time point of the plurality of motion time points to the angle of each lower leg of the subject with respect to the ground at the time when the subject is stationary, and
calculates an angle of each lower leg of the subject with respect to the ground at the grounding time by adding an integral value of the angular velocity acquired from a motion time point that is the closest to and immediately before the grounding time of the plurality of motion time points to the grounding time to a predetermined angle of each lower leg of the subject with respect to the ground at the first motion time point.

7. The information processing apparatus according to any one of claims 4 to 6, wherein

the controller
specifies an error relative to the angular velocity of the sagittal plane of each lower leg of the subject in the predetermined walking period so that an integral value of an angular velocity of the sagittal plane of each lower leg of the subject before a final time point of the predetermined walking period becomes zero, and calculates the angle of each lower leg of the subject with respect to the ground at the grounding time based on the specified error.

8. The information processing apparatus according to any one of claims 3 to 7, wherein

the controller
acquires information indicating a moving speed and a moving direction of the subject from a measuring instrument that measures the information indicating the moving speed and the moving direction of the subject, and sets a stable period in which the moving speed and the moving direction are constant as the predetermined walking period based on the acquired information indicating the moving speed and the moving direction.

9. The information processing apparatus according to any one of claims 3 to 8, wherein

the controller
acquires an angular velocity or an acceleration of the subject in a predetermined candidate period from a measuring instrument that measures the angular velocity or the acceleration of the subject, specifies a plurality of motion time points, in the predetermined candidate period, when a feature corresponding to one step of the subject appears in the angular velocity or the acceleration of the subject, and sets a stable period, in which an interval of motion time points is constant, in the predetermined candidate period as the predetermined walking period based on the plurality of motion time points that has been specified.

10. The information processing apparatus according to any one of claims 3 to 8, wherein

the controller
acquires an angular velocity or an acceleration of the subject in a predetermined candidate period from a measuring instrument that measures the angular velocity or the acceleration of the subject, specifies a plurality of motion time points, in the predetermined candidate period, when a feature corresponding to one step of the subject appears in the angular velocity or the acceleration of the subject, and sets a stable period, in which a feature quantity of an operation of the subject at the motion time point is constant, in the predetermined candidate period, as the predetermined walking period based on the plurality of motion time points that has been specified.

11. An information processing system comprising:

a measuring instrument; and
an information processing apparatus configured to communicate with the measuring instrument, wherein the measuring instrument
generates measured information regarding a movement of a subject, and the information processing apparatus
acquires the measured information from the measuring instrument, acquires information regarding a stride length of the subject and information regarding an angle of each lower leg of the subject with respect to a ground in a case where both feet of the subject are grounded based on the acquired measured information, and calculates at least any one of an angle of each knee joint of the subject and an angle of a hip joint of the subject that satisfy a geometric constraint derived from a structure of lower limbs of the subject in a case where both feet of the subject are grounded based on the acquired information regarding the stride length and the angle.

12. An information processing method causing a computer to execute processing for:

**EP 3 649 938 A1**

acquiring information regarding a stride length of a subject and information regarding an angle of each lower leg of the subject with respect to a ground in a case where both feet of the subject are grounded; and calculating at least any one of an angle of each knee joint of the subject and an angle of a hip joint of the subject that satisfy a geometric constraint derived from a structure of lower limbs of the subject in a case where both feet of the subject are grounded based on the acquired information regarding the stride length and the angle.

# FIG. 1

[RELATIONSHIP BETWEEN STRIDE LENGTH AND LOWER LIMBS AT THE TIME WHEN BOTH LEGS ARE GROUNDED DURING WALKING]

LEFT LEG

IT IS ASSUMED THAT "LENGTH OF THIGH" BE EQUAL TO "LENGTH OF LOWER LEG"

RIGHT LEG

100

110

L

$\varphi$

$\alpha$

$\beta$

$\theta_L$

$\theta_R$

SL

HIP JOINT

KNEE JOINT

THIGH (UPPER THAN KNEE)

LOWER LEG (LOWER THAN KNEE)

FOOT (FRONT SIDE OF ANKLE)

LOWER LIMB

# FIG. 2

# FIG. 3

# FIG. 4

# FIG. 5

# FIG. 6

(6-1)
<TENDENCY OF POSITION INFORMATION>

MOVE STRAIGHT AND
EQUAL INTERVALS

START    TRAVELING DIRECTION    END

MOVEMENTS OF
STEPS ARE SIMILAR
TO EACH OTHER

(6-2)
<GYRO DATA>

(6-3) [RELATIONSHIP BETWEEN STRIDE LENGTH AND LOWER LIMBS AT
THE TIME WHEN BOTH LEGS ARE GROUNDED DURING WALKING]

LEFT LEG

$L$

$\varphi$

IT IS ASSUMED THAT
"LENGTH OF THIGH" BE
EQUAL TO "LENGTH OF
LOWER LEG"

RIGHT LEG

$\beta$

$\alpha$

$\theta_L$

$\theta_R$

SL

CALCULATE STRIDE LENGTH BY
DIVIDING MOVEMENT DISTANCE
BY THE NUMBER OF STEPS

CALCULATE ANGLES $\theta_L$ AND $\theta_R$
OF LEFT AND RIGHT SHINS BY
INTEGRATING GYRO

# FIG. 7

# FIG. 8

# FIG. 9

900

MOVE
STRAIGHT
AND EQUAL
INTERVALS

TRAVELING
DIRECTION

START

END

→STRIDE LENGTH TENDS TO BE CONSTANT

901

FREQUENTLY
TURN

INTERVALS
VARY

END

START

→STRIDE LENGTH TENDS TO VARY

# FIG. 10

PATTERNS OF FOUR
STEPS ARE SIMILAR
TO EACH OTHER

1000

1001

PATTERN CHANGES IN
THE MIDDLE

ANGULAR
VELOCITY

TIME

ANGULAR
VELOCITY

TIME

→STRIDE LENGTH TENDS TO BE CONSTANT

→STRIDE LENGTH TENDS TO VARY

# FIG. 11

# FIG. 12

# FIG. 13

RIGHT SHIN ANGLE

LEFT SHIN ANGLE

1300

SHIN ANGLE (LEFT LEG)

π

0

-π

No.1 No.2 No.3 No.4

BOTH-LEG
GROUNDING PERIOD

TIME

1301

| PERIOD NUMBER | TIME POINT | LEFT SHIN ANGLE $\theta_L$ | RIGHT SHIN ANGLE $\theta_R$ |
|---|---|---|---|
| No1 | 1 | 103 | 30 |
| No1 | 2 | 102 | 28 |
| No1 | 3 | 105 | 25 |
| No1 | 4 | 110 | 19 |
| … | … | … | … |

# FIG. 14

POCKET

201

RIGHT SHIN

201

LEFT SHIN

201

SENSOR DEVICE

# FIG. 15

SAGITTAL PLANE    TRAVERSE PLANE    CORONAL PLANE

SAGITTAL PLANE
ANGULAR VELOCITY
(POSITIVE)

VERTICAL
ACCELERATION

# FIG. 16

POSITIONING
SENSOR

201

SUBJECT

# FIG. 17

FIXED DISTANCE (10 m)

FIXED WALKING COURSE

201

START POSITION

END POSITION

PLEASE INPUT DISTANCE YOU WALKED

○ 5 m

◉ 10 m

○ 20 m

TRANSMIT

# FIG. 18

1800

| TIME POINT | ANGULAR VELOCITY (SAGITTAL PLANE DIRECTION) | ACCELERATION (VERTICAL DIRECTION) |
|---|---|---|
| 1458191010.3 | 2.4 | 0.2 |
| 1458191010.4 | 2.3 | 0.8 |
| 1458191010.5 | 10.9 | 4.3 |
| 1458191010.6 | 27.9 | 6.3 |
| … | … | … |
| 1458195162.4 | 1.9 | 0.1 |

# FIG. 19

1900

| TIME POINT | ANGULAR VELOCITY (SAGITTAL PLANE DIRECTION) | ACCELERATION (VERTICAL DIRECTION) |
|---|---|---|
| 1458191011.8 | 0.9 | 1.3 |
| 1458191011.9 | 6.8 | 1.0 |
| 1458191012.0 | 30.8 | 7.8 |
| 1458191012.1 | 56.4 | 8.8 |
| ... | ... | ... |
| 1458195161.1 | 1.0 | 0.1 |

# FIG. 20

2000

| TIME POINT | LATITUDE | LONGITUDE | CERTAINTY |
|------------|----------|-----------|-----------|
| 1458191015 | 37.656 | 137.988 | 0.73 |
| 1458191020 | 37.656 | 137.987 | 0.65 |
| … | … | … | … |
| 1458195160 | 37.647 | 137.980 | 0.99 |

# FIG. 21

SHIFT WIDTH X

WINDOW WIDTH W

WINDOW START POINT

WINDOW END POINT

CANDIDATE PERIOD OBTAINED AS RESULT OF COMBINING CONSECUTIVE CANDIDATE WINDOWS

# FIG. 22

2200

| CANDIDATE PERIOD NUMBER | START POINT | END POINT |
|---|---|---|
| 1 | 1458191012.7 | 1458191082.9 |
| 2 | 1458191222.0 | 1458191534.8 |
| ... | ... | ... |
| N | 1458194816.8 | 1458194997.0 |

# FIG. 23

1800

| TIME POINT | ANGULAR VELOCITY (SAGITTAL PLANE DIRECTION) | ACCELERATION (VERTICAL DIRECTION) |
|---|---|---|
| 1458191010.3 | 2.4 | 0.2 |
| 1458191010.4 | 2.3 | 0.8 |
| … | … | … |
| 1458191012.7 | 3.5 | 0.3 |
| 1458191012.8 | 2.7 | 0.1 |
| … | … | … |
| 1458191084.9 | 0.9 | 0.5 |
| … | … | … |
| 1458195162.4 | 1.9 | 0.1 |

START POINT OF CANDIDATE NUMBER 1

END POINT OF CANDIDATE NUMBER 1

# FIG. 24

| PEAK NUMBER | PEAK TIME POINT |
|---|---|
| 1 | 1458191014.0 |
| 2 | 1458191015.4 |
| … | … |
| NL | 1458191082.8 |

2400

| PEAK NUMBER | PEAK TIME POINT |
|---|---|
| 1 | 1458191014.7 |
| 2 | 1458191016.0 |
| … | … |
| NR | 1458191082.0 |

2401

2402

| PEAK NUMBER | PEAK TIME POINT | WHICH FOOT |
|---|---|---|
| 1 | 1458191014.0 | LEFT |
| 2 | 1458191014.7 | RIGHT |
| 3 | 1458191015.4 | LEFT |
| 4 | 1458191016.0 | RIGHT |
| … | … | … |
| N-1 | 1458191082.0 | LEFT |
| N | 1458191082.8 | RIGHT |

# FIG. 25

2500

| PEAK NUMBER | PEAK HEIGHT | PEAK INTERVAL |
|:-----------:|:-----------:|:-------------:|
| 1 | 203.4 | 0.7 |
| 2 | 323.5 | 0.7 |
| 3 | 343.5 | 0.7 |
| 4 | 335.7 | 0.6 |
| … | … | … |
| N-1 | 298.0 | 0.8 |
| N | 186.4 | --- |

FIG. 26

2000

| TIME POINT | LATITUDE | LONGITUDE | CERTAINTY |
|---|---|---|---|
| 1458191015 | 37.656 | 137.988 | 0.73 |
| 1458191020 | 37.656 | 137.987 | 0.65 |
| … | … | … | … |
| 1458191080 | 37.625 | 137.944 | 0.87 |
| … | … | … | … |
| 1458195160 | 37.647 | 137.980 | 0.99 |

IMMEDIATELY AFTER START POINT OF CANDIDATE NUMBER THAT IS DETERMINED AS STABLE WALKING PERIOD

IMMEDIATELY BEFORE END POINT OF CANDIDATE NUMBER THAT IS DETERMINED AS STABLE WALKING PERIOD

# FIG. 27

1900

| TIME POINT | ANGULAR VELOCITY (SAGITTAL PLANE DIRECTION) | ACCELERATION (VERTICAL DIRECTION) |
|---|---|---|
| 1458191010.3 | 2.4 | 0.2 |
| 1458191010.4 | 2.3 | 0.8 |
| … | … | … |
| 1458191034.5 | 324.0 | 12.1 |
| … | … | … |
| 1458191035.2 | -34.6 | -5.1 |
| … | … | … |
| 1458195162.4 | 1.9 | 0.1 |

OCCURRENCE POINT OF
CURRENT TARGET PEAK POINT

CERTAIN PERIOD OF TIME
(=0.7 SECONDS)

# FIG. 28

1900

| TIME POINT | ANGULAR VELOCITY (SAGITTAL PLANE DIRECTION) | ACCELERATION (VERTICAL DIRECTION) |
|---|---|---|
| 1458191010.3 | 2.4 | 0.2 |
| 1458191010.4 | 2.3 | 0.8 |
| … | … | … |
| 1458191033.5 | 2.7 | -4.7 |
| … | … | … |
| 1458191034.5 | 324.0 | 12.1 |
| … | … | … |
| 1458195162.4 | 1.9 | 0.1 |

CERTAIN PERIOD OF TIME (=ONE SECOND)

OCCURRENCE POINT OF CURRENT TARGET PEAK POINT

# FIG. 29

2900

| GROUNDING PERIOD NUMBER | START POINT (HEEL-STRIKE POINT) | END POINT (TOE-OFF POINT) |
|---|---|---|
| 1 | 1458191013.2 | 1458191013.3 |
| 2 | 1458191013.8 | 1458191013.9 |
| … | … | … |
| N-1 | 1458191082.2 | 1458191082.3 |

# FIG. 30

3000

| TIME POINT | LEFT LEG SHIN ANGLE ΘL(t) | RIGHT LEG SHIN ANGLE ΘL(t) | |
|---|---|---|---|
| 1458191012.7 | 0.00 | 0.00 | ← START POINT OF CANDIDATE NUMBER 1 |
| 1458191012.8 | 0.31 | 0.12 | |
| … | … | … | |
| 1458191084.9 | 0.11 | 0.08 | ← END POINT OF CANDIDATE NUMBER 1 |

# FIG. 31

# FIG. 32

ONE LEG

ACCELERATION
VERTICAL COMPONENT

ACCELERATION
OF GRAVITY

$\theta_0$

FIG. 33

3300

| GROUNDING PERIOD NUMBER | START POINT (HEEL-STRIKE POINT) | END POINT (TOE-OFF POINT) | TIME POINT | RIGHT LEG SHIN ANGLE (°) | LEFT LEG SHIN ANGLE (°) |
|---|---|---|---|---|---|
| 1 | 1458191013.2 | 1458191013.3 | 1458191013.201 | 34.2 | -35.7 |
| | | | 1458191013.211 | 30.1 | -27.8 |
| | | | … | … | … |
| | | | 1458191013.298 | -37.5 | 40.1 |
| … | … | … | … | … | … |
| N-1 | 1458191082.2 | 1458191082.3 | … | … | … |

FIG. 34

3300

| GROUNDING PERIOD NUMBER | START POINT (HEEL-STRIKE POINT) | END POINT (TOE-OFF POINT) | TIME POINT | RIGHT LEG SHIN ANGLE (°) | LEFT LEG SHIN ANGLE (°) | STRIDE LENGTH (cm) |
|---|---|---|---|---|---|---|
| 1 | 1458191013.2 | 1458191013.3 | 1458191013.201 | 34.2 | -35.7 | 102.3 |
| | | | 1458191013.211 | 30.1 | -27.8 | |
| | | | … | … | … | |
| | | | 1458191013.298 | -37.5 | 40.1 | |
| … | … | … | … | … | … | … |
| N-1 | 1458191082.2 | 1458191082.3 | … | … | … | … |

FIG. 35

3500

| GROUNDING PERIOD NUMBER | TIME POINT | RIGHT LEG SHIN ANGLE (°) | LEFT LEG SHIN ANGLE (°) | STRIDE LENGTH (cm) | KNEE JOINT ANGLE β OF LANDING LEG(°) | KNEE JOINT ANGLE α OF ANOTHER LEG(°) | HIP JOINT ANGLE Φ (°) |
|---|---|---|---|---|---|---|---|
| 1 | 1458191013.201 | 34.2 | -35.7 | 102.3 | 154.1 | 155.9 | 85.4 |
| | 1458191013.211 | 30.1 | -27.8 | | 160.7 | 168.9 | 83.3 |
| | … | … | … | | … | … | … |
| | 1458191013.298 | -37.5 | 40.1 | | 143.0 | 151.1 | 97.8 |
| … | … | … | … | … | … | … | … |
| N-1 | … | … | … | … | … | … | … |

# FIG. 36

3500

| WALKING PERIOD NUMBER | GROUNDING PERIOD NUMBER | TIME POINT | RIGHT LEG SHIN ANGLE (°) | LEFT LEG SHIN ANGLE (°) | STRIDE LENGTH (cm) | KNEE JOINT ANGLE $\beta$ OF LANDING LEG(°) | KNEE JOINT ANGLE $\alpha$ OF ANOTHER LEG(°) | HIP JOINT ANGLE $\Phi$(°) |
|---|---|---|---|---|---|---|---|---|
| 1 | 1 | 1458191013.201 | 34.2 | -35.7 | 102.3 | 154.1 | 155.9 | 85.4 |
| | | 1458191013.211 | 30.1 | -27.8 | | 160.7 | 168.9 | 83.3 |
| | | ... | ... | ... | | ... | ... | ... |
| | | 1458191013.298 | -37.5 | 40.1 | | 143.0 | 151.1 | 97.8 |
| | ... | ... | ... | ... | ... | ... | ... | ... |
| | N1-1 | ... | ... | ... | ... | ... | ... | ... |
| 2 | 1 | 1458191222.885 | 36.7 | -35.2 | 104.5 | 153.1 | 150.9 | 85.1 |
| | | 1458191223.114 | 31.7 | -20.2 | | 160.9 | 167.9 | 90.1 |
| | | ... | ... | ... | | ... | ... | ... |
| | | 1458191013.298 | -32.0 | 40.0 | | 140.0 | 136.5 | 95.2 |
| | ... | ... | ... | ... | ... | ... | ... | ... |
| | N2-1 | ... | ... | ... | ... | ... | ... | ... |
| ... | ... | ... | ... | ... | ... | ... | ... | ... |

# FIG. 37

# FIG. 38

# FIG. 39A

START

ACQUIRE SENSOR DATA FOR FIXED PERIOD — S3901

SPECIFY ONE OR MORE CANDIDATE PERIODS THAT MAY INCLUDE WALKING PERIOD — S3902

SELECT ANY ONE OF ONE OR MORE CANDIDATE PERIODS — S3903

SPECIFY WALKING PERIOD IN CANDIDATE PERIOD — S3904

CALCULATE EVALUATION VALUE OF STABLE WALKING ABILITY IN WALKING PERIOD — S3905

IS EVALUATION VALUE OF STABLE WALKING ABILITY EQUAL TO OR MORE THAN THRESHOLD? — S3906

NO

YES

CALCULATE EVALUATION VALUE OF STABLE MOVING ABILITY IN WALKING PERIOD — S3907

IS EVALUATION VALUE OF STABLE MOVING ABILITY EQUAL TO OR MORE THAN THRESHOLD? — S3908

NO

YES

A

B

# FIG. 39B

Ⓐ

| SPECIFY BOTH-LEG GROUNDING PERIOD IN WALKING PERIOD | S3909 |

| CALCULATE SHIN ANGLE OF EACH LEG IN BOTH-LEG GROUNDING PERIOD | S3910 |

| CALCULATE STRIDE LENGTH IN WALKING PERIOD | S3911 |

| CALCULATE ANGLE OF KNEE JOINT AND ANGLE OF HIP JOINT | S3912 |

Ⓑ ← NO — SELECT ALL OF ONE OR MORE CANDIDATE PERIODS? — S3913

YES

END

## INTERNATIONAL SEARCH REPORT

| | International application No. |
|---|---|
| | PCT/JP2017/024561 |

| A. CLASSIFICATION OF SUBJECT MATTER |
|---|
| A61B5/11(2006.01)i |

According to International Patent Classification (IPC) or to both national classification and IPC

| B. FIELDS SEARCHED |
|---|

Minimum documentation searched (classification system followed by classification symbols)
A61B5/11

Documentation searched other than minimum documentation to the extent that such documents are included in the fields searched
Jitsuyo Shinan Koho          1922–1996    Jitsuyo Shinan Toroku Koho    1996–2017
Kokai Jitsuyo Shinan Koho    1971–2017    Toroku Jitsuyo Shinan Koho    1994–2017

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| A | JP 2009-125270 A (Panasonic Electric Works Co., Ltd.), 11 June 2009 (11.06.2009), (Family: none) | 1-12 |
| A | JP 2012-065723 A (Dainippon Printing Co., Ltd.), 05 April 2012 (05.04.2012), (Family: none) | 1-12 |

☐ Further documents are listed in the continuation of Box C.  ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13 September 2017 (13.09.17) | 26 September 2017 (26.09.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

**EP 3 649 938 A1**

**REFERENCES CITED IN THE DESCRIPTION**

**Patent documents cited in the description**

- JP 2014208257 A

- JP 2012065723 A